# EUROPEAN PATENT APPLICATION

(11) **EP 1 577 300 A1**
(43) Date of publication of application: **21.09.2005**
(21) Application number: 03756678.3
(22) Date of filing: 17.10.2003
(51) Int. Cl.: C07D 213/53, C07D 213/06, C07D 213/30, C07D 213/26, C07D 213/22, C09K 11/06, H05B 33/14, C07D 409/14

(54) **PLATINUM COMPLEXES**

(30) Priority: 01.11.2002 JP 2002320455; 04.03.2003 JP 2003057603; 25.03.2003 JP 2003083035
(71) Applicant: Takasago International Corporation, Tokyo-to 144-8721 (JP)
(72) Inventor: ITOH, Hisanori, Hiratsuka-shi, Kanagawa 254-00 (JP); NAKAYAMA, Yuji, Hiratsuka-shi, Kanagawa 254-00 (JP); MATSUSHIMA, Yoshimasa, Hiratsuka-shi, Kanagawa 254-00 (JP); HORI, Yoji, Hiratsuka-shi, Kanagawa 254-00 (JP)
(74) Representative: Cresswell, Thomas Anthony
(86) International application number: PCT/JP2003/013317
(87) International publication number: WO 2004/039781

(57) **Abstract**

The invention aims at providing platinum complexes useful as materials for light emitting devices and extremely excellent in heat stability, luminous characteristics, and luminous efficiency, and a process for effective preparation thereof. The invention relates to platinum complexes represented by the general formula [1]: 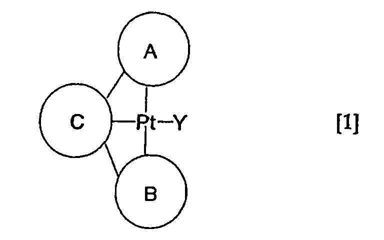 wherein any two of A, B and C are each independently an optionally substituted nitrogenous aromatic heterocyclic group and the other is optionally substituted aryl or optionally substituted heteroaryl; and Y is halogeno or an optionally substituted aryl or heteroaryl group which is bonded either directly or through oxygen (-O-) or sulfur (-S-) (with the proviso that when the adjacent two rings are nitrogenous aromatic heterocyclic groups, the cases wherein Y is chloro are excepted, while when the nonadjacent two rings are nitrogenous aromatic heterocyclic groups, the cases wherein Y is not halogeno are excepted).

## Description

### Technical Field

The present invention relates to a novel platinum complex useful as a material for a light emitting device capable of converting an electric energy into light, and emitting light. The platinum complex of the invention is useful as a novel light emitting material preferably usable in the field of a display device, a display, a backlight, an electrophotography, an illumination light source, a recording light source, an exposure light source, a reading light source, an indicator, a signboard, an interior product, or the like.

### Background Art

Nowadays, the study and development on various display devices have been made actively. Especially, an organic electroluminescent device (which is hereinafter referred to as an organic EL device) has received attention as a promising display device because it is capable of providing high luminescence at a low voltage. For example, a light emitting device obtained by forming an organic thin film by vapor deposition of an organic compound is known. For this light emitting device, a tris(8-hydroxyquinolinato)aluminum complex (which is hereinafter referred to as Alq) which is a fluorescent material is used as an electron transport material, and laminated with a hole transport material (such as an amine compound), thereby to largely improve the luminous characteristics as compared with a conventional monolayer type device.

Thus, the move to apply such an organic EL device to a multicolor display has been studied increasingly in recent years. However, in order to develop a high-performance multicolor display, it is necessary to improve the characteristics of light emitting devices of respective colors of red, green, and blue which are the three primary colors of light, and the efficiencies thereof.

As a means for improving the characteristics of the light emitting device, it has been also proposed that a phosphorescent material is used other than the fluorescent material for the organic EL device light emitting layer. In general, the light emitting process of phosphorescence is the process in which molecules are excited from the ground state to the excited state, and subsequently nonradiative transition called intersystem crossing occurs from the singlet excited state to the triplet excited state. Phosphorescence shows luminescence of the ground state from the triplet state. High luminous efficiency can be presumably achieved when the singlet state and the triplet state of an organic phosphorescent material are utilized. This conceivably contributes to the increase in life of the organic EL device.

As the organic EL devices using such phosphorescent materials, green light emitting devices utilizing phosphorescence from a tris(2-phenylpyridine)iridium complex (Ir(ppy)₃) which is an orthometalated iridium complex has been reported (Applied Physics Letters, 75, 4 (1999)).

Further, (2,3,7,8,12,13,17,18-octaethyl-21H, 23H-porphyrinato-N,N,N,N)platinum (Pt(OEP)) which is an orthometalated platinum complex has been also reported (USP 6,303,238: M. A. Baldo et al., Nature, 395 (1998) 151-154). This platinum complex is a red phosphorescent material showing a value of as high as 4 % in terms of an external quantum efficiency. However, a still further efficient phosphorescent material has been demanded.

Further, attention is focused on a light emitting device using a platinum complex, and then, a bis (2-phenylpyridine) platinum complex Pt (ppy)₂, and analogous products have been reported.

As the platinum complexes, other than this, (6-phenyl-2,2'-bipyridinato-C,N,N)platinum chloride (II), and derivatives thereof have been reported (E. C. Constable et al., J. Chem. Soc. Dalton Trans., 1990, 443-449; Tsz-Chun Cheung et al., J. Chem. Soc. Dalton Trans., 1996, 1645-1651; Yurngdong Jahng et al., Inorganica Chimica Acta, 267 (1998) 265-270; Siu-Wai Lai et al., Inorg. Chem., 38 (1999) 4046-4055). It is reported that the platinum complexes show a UV absorption phenomenon and a luminescent phenomenon. A study has been reported to use a substance obtained by allowing polyethylene glycol to carry the platinum complex for protein analysis in vivo by utilizing this property (Chi-Ming Che et al., Chem. Commun. 2002, 2556-2557). However, up to now, the application of the platinum complex to a light emitting device such as an organic EL device has not been reported in any document. This is presumably for the following reason. Only the fact that the platinum complex has a UV absorption phenomenon and a luminescent phenomenon does not mean that it can be used for an organic EL device or the like. Further, even when it can be used for an organic EL device, whether the resulting device is better than the currently and commonly used organic EL devices or not cannot be predicted with ease.

Whereas, although there is no description on the luminescence, there has been a report that a 1,3-di(2-pyridyl)phenyl platinum chloride dihydrate was synthesized as a synthesis example of a platinum complex (organometallics 1999, 18, 3337-3341).

Further, in JP-A-2002-175884, although the data of the external quantum efficiency or the like is not described, a platinum complex in which two nitrogen atom-containing cyclic groups and two carbon atom-containing cyclic groups are coordinated is disclosed as a metal coordination compound for a light emitting device.

The platinum complexes herein specifically disclosed include three patterns such as the one in which all the four cyclic groups each independently coordinate to platinum; the one in which with only two of the four cyclic groups being bonded, respective ones coordinate to platinum; and the one in which with the four cyclic groups being bonded in a group of two, respective ones coordinate to platinum. However, the complex in which with three or four of the cyclic groups being bonded, respective ones coordinate to platinum has not been disclosed.

On the other hand, all the production methods described in the documents, of the (6-phenyl-2,2'-bipyridinato-C,N,N)platinum chloride (II) (which is hereinafter abbreviated as a [PtL (C1) ] complex) and derivatives thereof are the methods in which K₂PtCl₄ is used as a platinum source, and the reaction is effected in an acetonitrile / water solvent. In the case of an unsubstituted [PtL (Cl)] complex, and the case where the substituent of a phenyl group or a pyridyl group is a hydrocarbon group, an acceptable yield can be obtained. However, in other cases, a satisfactory yield is not necessarily obtained.

In recent years, various substances having fluorescence have been used for dyes for filters, color conversion filters, dyes for photographic materials, sensitizing dyes, dyes for pulp dyeing, laser dyes, fluorescent drugs for medical diagnosis, materials for organic light emitting devices, and the like. They are in increasing demand, and there is a demand for the development of novel light emitting materials.

In the organic light emitting devices, the one which has achieved high luminescence is a device obtained by stacking an organic substance by vacuum evaporation. However, fabrication of the device by a coating method is desirable from the viewpoints of the simplification of the manufacturing process, the workability, the increase in area, and the like. However, the devices fabricated by a conventional coating method are inferior particularly in luminous efficiency to the devices fabricated by a vapor deposition method. Also from such viewpoints, there has been a demand for the development of a novel light emitting material.

As described above, various studies have been made toward the practical utilization of the next-generation display devices. Especially, the organic EL device using a phosphorescent material has been particularly in the limelight from the viewpoint of the improvement of the characteristics of the device. However, the study has been just started, and there are many problems of the luminous characteristics, the luminous efficiency, color purity, and the optimization of the structure of the device, and the like. In order to solve these problems, in actuality, the development of a novel phosphorescent material, and further the development of the efficient production method of the material have been demanded.

### Disclosure of the Invention

The present invention has been made in view of the foregoing circumstances. It is an object of the invention to provide a platinum complex useful as, for example, a material for a light emitting device, and very excellent in heat stability, luminous characteristics, and luminous efficiency, and an effective production method thereof.

### Brief Description of the Drawings

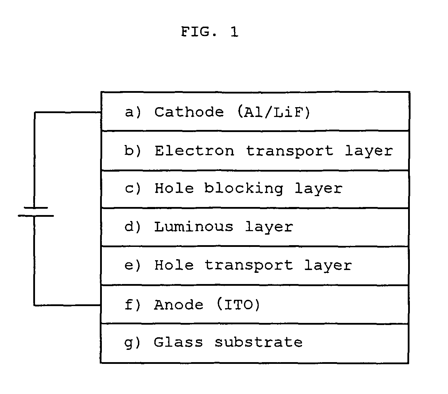

FIG. 1 is a diagram showing an example of a configuration of an organic EL device using a platinum complex of the present invention.

The reference characters in FIG. 1 will be described below.
(a) Second electrode (metal electrode, cathode)
(b) Electron transport layer
(c) Hole blocking layer
(d) Luminous layer (host material and dopant)
(e) Hole transport layer
(f) First electrode (transparent electrode, anode)
(g) Glass substrate

### Best Mode for Carrying Out the Invention

The present invention relates to a platinum complex represented by the following general formula [1]: 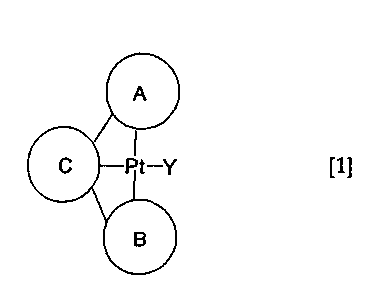 [where in the formula, any two of a ring A, a ring B, and a ring C each independently represent a nitrogen-containing aromatic heterocyclic group coordinating to a platinum atom through a nitrogen atom, which may be substituted, and the other represents an aryl group which may be substituted, or a heteroaryl group which may be substituted, and Y represents a halogen atom, or an aryl group which may be substituted, or a heteroaryl group which may be substituted, bonded directly or through an oxygen atom (-O-) or a sulfur atom (-S-) (provided that when the adjacent two rings are nitrogen-containing aromatic heterocyclic groups, the cases where Y is a chlorine atom are excluded, and that when the nonadjacent two rings are nitrogen-containing aromatic heterocyclic groups, the cases where Y is a group other than a halogen atom are excluded)].

Preferred examples of the platinum complex represented by the general formula [1] may include the platinum complexes represented by the following general formula [1']: 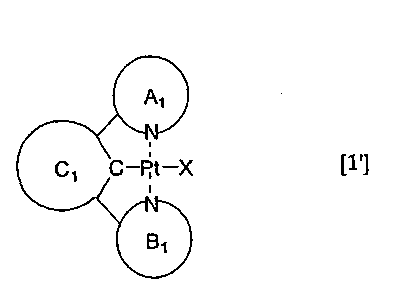 (where in the formula, a ring A₁ and a ring B₁ each independently represent a nitrogen-containing aromatic heterocyclic group which may be substituted, a ring C₁ represents an aryl group which may be substituted or a heteroaryl group which may be substituted, and X represents a halogen atom.)

Preferred examples of the platinum complex represented by the general formula [1'] may include the platinum complexes represented by the following general formula [1a'] 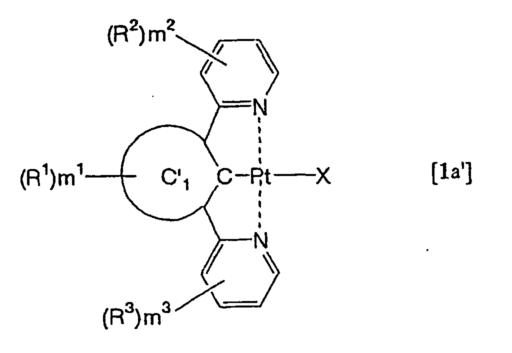 (where in the formula, a ring C'₁ represents an aryl group or a heteroaryl group; R¹, R², and R³ each independently represent a hydrogen atom, an alkyl group, a haloalkyl group, an aralkyl group, an alkenyl group, an alkynyl group, an aryl group, an amino group, a mono- or di-alkylamino group, a mono- or di-arylamino group, an alkoxy group, an aryloxy group, a heteroaryloxy group, an alkoxycarbonyl group, an acyloxy group, an acylamino group, an alkoxycarbonylamino group, an aryloxycarbonylamino group, a sulfonylamino group, a sulfamoyl group, a carbamoyl group, an alkylthio group, an arylthio group, a heteroarylthio group, a sulfonyl group, a sulfinyl group, an ureido group, a phosphoramide group, a hydroxyl group, a mercapto group, a halogen atom, a cyano group, a sulfo group, a carboxyl group, a nitro group, a hydroxamic acid group, a sulfino group, a hydrazino group, an aliphatic heterocyclic group, an aromatic heterocyclic group, a substituted silyl group, or a polymerizable group; further, a plurality of R¹s, a plurality of R²s, or/and a plurality of R³s may together form a fused ring with pyridine rings or the ring C to which they are bonded; X represents a halogen atom; m¹, m², and m³ represent the numbers of the substituents R¹, R², and R³, respectively, and m¹ represents an integer of 0 to 3, and m² and m³ each represent an integer of 0 to 4; and further, when m¹, m², and m³ are each an integer of 2 or more, a plurality of R¹s, a plurality of R²s, and a plurality of R³s may be mutually the same or different.)

More preferred examples of the platinum complex represented by the general formula [1'] may include the platinum complexes represented by the general formula [1b'] 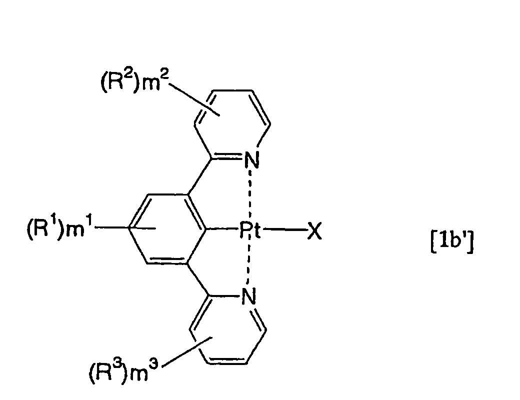 (where in the formula, R¹, R², R³, X, m¹, m², and m³ represent the same meanings as described above.)

Other preferred examples of the platinum complex represented by the general formula [1] may include the platinum complexes represented by the following general formula [1"]: 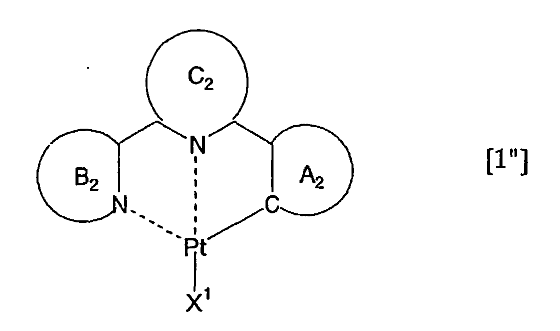 (where in the formula, a ring B₂ and a ring C₂ each independently represent a nitrogen-containing aromatic heterocyclic group which may be substituted, a ring A₂ represents an aryl group which may be substituted or a heteroaryl group which may be substituted; further, the ring B₂ and the ring C₂, and the ring C₂ and the ring A₂, or the ring B₂, the ring C₂, and the ring A₂ may be mutually bonded to form a fused ring; and X¹ represents a fluorine atom, a bromine atom, or an iodine atom.)

Preferred examples of the platinum complex represented by the general formula [1''] may include the platinum complexes represented by the following general formula [1a''] 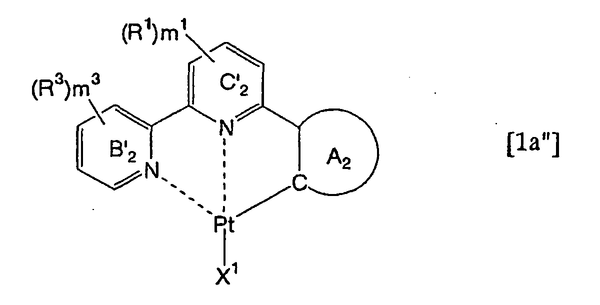 (where in the formula, a ring A₂ represents an aryl group which may be substituted or a heteroaryl group which may be substituted; R¹ and R³ each independently represent a hydrogen atom, an alkyl group, a haloalkyl group, an aralkyl group, an alkenyl group, an alkynyl group, an aryl group, an amino group, a mono- or di-alkylamino group, a mono- or di-arylamino group, an alkoxy group, an aryloxy group, a heteroaryloxy group, an alkoxycarbonyl group, an acyloxy group, an acylamino group, an alkoxycarbonylamino group, an aryloxycarbonylamino group, a sulfonylamino group, a sulfamoyl group, a carbamoyl group, an alkylthio group, an arylthio group, a heteroarylthio group, a sulfonyl group, a sulfinyl group, an ureido group, a phosphoramide group, a hydroxyl group, a mercapto group, a halogen atom, a cyano group, a sulfo group, a carboxyl group, a nitro group, a hydroxamic acid group, a sulfino group, a hydrazino group, an aliphatic heterocyclic group, an aromatic heterocyclic group, a substituted silyl group, or a polymerizable group; further, a plurality of R¹s or/and a plurality of R³s may together form a fused ring with pyridine rings to which they are bonded; further, the ring B'₂ and the ring C'₂, and the ring C'₂ and the ring A₂, or the ring B'₂, the ring C'₂, and the ring A₂ may be mutually bonded to form a fused ring; m¹ and m³ represent the numbers of the substituents R¹ and R³, respectively, and m¹ represents an integer of 0 to 3, and m³ represents an integer of 0 to 4; and further, when m¹ and m³ each is an integer of 2 or more, a plurality of R¹s and a plurality of R³s may be mutually the same or different, and X¹ is the same as described above.)

More preferred examples of the platinum complex represented by the general formula [1] may include the platinum complexes represented by the general formula [1'''] 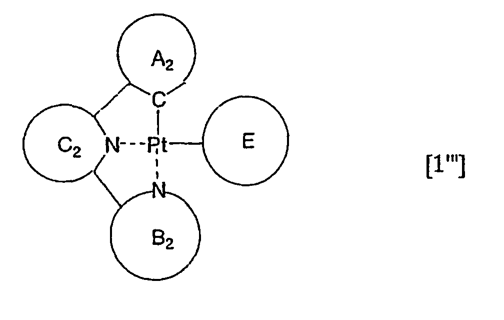 (where in the formula, a ring B₂ and a ring C₂ each independently represent a nitrogen-containing aromatic heterocyclic group which may be substituted, a ring A₂ and a ring E each independently represent an aryl group which may be substituted, or a heteroaryl group which may be substituted; the ring A₂ and the ring C₂, and the ring C₂ and the ring B₂, or the ring A₂, the ring C₂, and the ring B₂ may be mutually bonded to form a fused ring; further, in the case where the ring A₂, the ring B₂, the ring C₂ or/and the ring E each have a substituent, when the substituent is a substituent capable of coordinating or bonding to a metal, a metal atom may be coordinated or bonded through a coordinatable or bondable atom in the substituent.)

Preferred examples of the platinum complex represented by the general formula [1'''] may include the platinum complexes represented by the following general formula [1a"']: 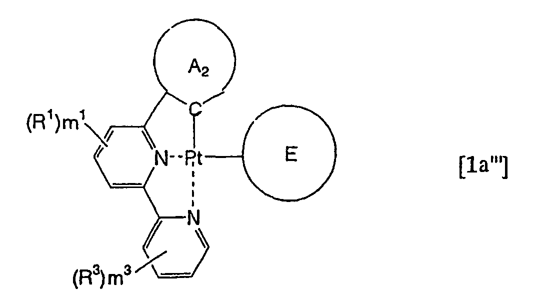 (where in the formula, the ring A₂ and the ring E are the same as described above; R¹ and R³ each independently represent an alkyl group, a haloalkyl group, an aralkyl group, an alkenyl group, an alkynyl group, an aryl group, an amino group, a mono- or di-alkylamino group, a mono- or di-arylamino group, an alkoxy group, an aryloxy group, a heteroaryloxy group, an alkoxycarbonyl group, an aryloxycarbonyl group, an acyloxy group, an acylamino group, an alkoxycarbonylamino group, an aryloxycarbonylamino group, a sulfonylamino group, a sulfamoyl group, a carbamoyl group, an alkylthio group, an arylthio group, a heteroarylthio group, a sulfonyl group, a sulfinyl group, an ureido group, a phosphoramide group, a hydroxyl group, a mercapto group, a halogen atom, a cyano group, a sulfo group, a carboxyl group, a nitro group, a hydroxamic acid group, a sulfino group, a hydrazino group, an aliphatic heterocyclic group, an aromatic heterocyclic group, a substituted silyl group, or a polymerizable group; further, R¹ and R³ may together form a fused ring with two pyridine rings to which they are bonded; R¹ and the ring A₂, or R¹, R³, and the ring A₂ may together form a fused ring; m¹ and m³ represent the numbers of the substituents R¹ and R³, respectively, m¹ represents an integer of 0 to 3, and m³ represents an integer of 0 to 4; and further, when m¹ and m³ are each an integer of 2 or more, a plurality of R¹s and a plurality of R³s may be mutually the same or different; further, a plurality of R¹s or/and a plurality of R³s may together form a fused ring with the pyridine rings to which they are bonded; and further when R¹, R³, and each substituent in the ring A₂ or/and the ring E are each a substituent capable of coordinating to a metal or capable of bonding to a metal, a metal atom may be coordinated or bonded through a coordinatable or bondable atom in the substituent.)

More preferred examples of the platinum complex represented by the general formula [1'''] may include the platinum complexes represented by the following general formula [1b''']: 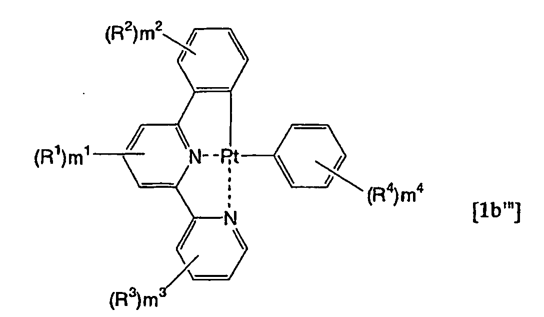 (where in the formula, R¹, R², R³, and R⁴ each independently represent an alkyl group, a haloalkyl group, an aralkyl group, an alkenyl group, an alkynyl group, an aryl group, an amino group, a mono- or di-alkylamino group, a mono- or di-arylamino group, an alkoxy group, an aryloxy group, a heteroaryloxy group, an alkoxycarbonyl group, an aryloxycarbonyl group, an acyloxy group, an acylamino group, an alkoxycarbonylamino group, an aryloxycarbonylamino group, a sulfonylamino group, a sulfamoyl group, a carbamoyl group, an alkylthio group, an arylthio group, a heteroarylthio group, a sulfonyl group, a sulfinyl group, an ureido group, a phosphoramide group, a hydroxyl group, a mercapto group, a halogen atom, a cyano group, a sulfo group, a carboxyl group, a nitro group, a hydroxamic acid group, a sulfino group, a hydrazino group, an aliphatic heterocyclic group, an aromatic heterocyclic group, a substituted silyl group, or a polymerizable group; further, R¹ and R², R¹ and R³, or/and R¹ and R² and R³ may together form a fused ring with the two pyridine rings, or the pyridine ring and the benzene ring to which they are bonded; m¹, m², m³, and m⁴ represent the numbers of the substituents R¹, R², R³, and R⁴, respectively, m¹ represents an integer of 0 to 3, m² and m³ each represent an integer of 0 to 4, and m⁴ represents an integer of 0 to 5; and further, when m¹, m², m³, and m⁴ are each an integer of 2 or more, a plurality of R¹s, a plurality of R²s, a plurality of R³s, and a plurality of R⁴s may be mutually the same or different; further, R¹s, R²s, R³s, or/and R⁴s may together form a fused ring with the pyridine rings or the benzene rings to which they are bonded; and further when R¹, R², R³, or/and R⁴ are each a substituent capable of coordinating to a metal or capable of bonding to a metal, a metal atom may be coordinated or bonded through a coordinatable or bondable atom in the substituent.)

The platinum complex represented by the general formula [1] of the invention is a platinum complex compound made of a tridentate ligand made of the ring A, the ring B, and the ring C, and a halogen atom, or an aryl group which may be substituted or a heteroaryl group which may be substituted, bonded directly or through an oxygen atom (-O-) or a sulfur atom (-S-) thereto (provided that when the adjacent two rings are nitrogen-containing aromatic heterocyclic groups, the cases where Y is a chlorine atom are excluded, and when the nonadjacent two rings are nitrogen-containing aromatic heterocyclic groups, the cases where Y is a group other than a halogen atom are excluded).

Herein, any two of the ring A, the ring B, and the ring C are the rings coordinating to a platinum atom through a nitrogen atom, and the residual one is the ring group bonding to a platinum atom through a carbon atom.

These rings may be each a monocyclic ring or a polycyclic ring, or a fused ring. Further, it is also acceptable that the ring A and the ring C, or the ring C and the ring B form a fused ring, or that the ring A, the ring C, and the ring B form a fused ring.

The platinum complexes are orthometalated platinum complexes.

Incidentally, the term "orthometalated complexes" is the generic name of the compound group described in, for example, YUUKI KINZOKU KAGAKU -KISO TO OUYOU- (Organometallic Chemistry -Principles and Applications-) written by Akio Yamamoto, p.p. 150 to 232, SHOKABO PUBLISHING Co., Ltd., issued in 1982, or Photochemistry and Photophysics of coordination Compounds, written by H. Yersin, p.p. 71 to 77, p.p. 135 to 146, Springer-Verlag Co., issued in 1987.

As each nitrogen-containing aromatic heterocyclic group which may be substituted, represented by the ring A, the ring B, and the ring C in the general formula [1], each nitrogen-containing aromatic heterocyclic group which may be substituted, represented by the ring A₁ and the ring B₁ in the general formula [1'], and each nitrogen-containing aromatic heterocyclic group which may be substituted, represented by the ring B₂ and the ring C₂ in the general formulae [1''] and [1'''], each independently, mention may be made of a nitrogen-containing aromatic heterocyclic group and a substituted nitrogen-containing aromatic heterocyclic group.

The nitrogen-containing aromatic heterocyclic group is, for example, a heterocyclic group having 2 to 15 carbon atoms, and at least one nitrogen atom as a hetero atom, and it may further have 1 to 3 hetero atoms such as nitrogen atoms, oxygen atoms, or sulfur atoms. Further, the nitrogen-containing aromatic heterocyclic group is a 5 to 8-membered, preferably 5- or 6-membered monocyclic nitrogen-containing aromatic heterocyclic group, or polycyclic or fused ring nitrogen containing aromatic heterocyclic group.

Specific examples of the nitrogen-containing aromatic heterocyclic ring may include a pyridine ring, a pyrimidine ring, a pyrazine ring, a pyridazine ring, a pyrazole ring, an imidazole ring, an oxazole ring, a thiazole ring, a quinoline ring, an isoquinoline ring, a quinoxaline ring, a phthalazine ring, a quinazole ring, a naphthyridine ring, a cinnoline ring, a benzimidazole ring, a benzoxazole ring, and a benzothiazole ring.

As the substituted nitrogen-containing aromatic heterocyclic group, mention may be made of a nitrogen-containing aromatic heterocyclic ring obtained by substituting at least one hydrogen atom of the nitrogen-containing aromatic heterocyclic group with a substituent. As the substituent, mention may be made of a hydrocarbon group, a substituted hydrocarbon group, an aliphatic heterocyclic group, a substituted aliphatic heterocyclic group, an aromatic heterocyclic group, a substituted aromatic heterocyclic group, an alkoxy group, a substituted alkoxy group, an aryloxy group, a substituted aryloxy group, an aralkyloxy group, a substituted aralkyloxy group, a heteroaryloxy group, a substituted heteroaryloxy group, an alkoxycarbonyl group, an aryloxycarbonyl group, an aralkyloxycarbonyl group, an acyl group, an acyloxy group, an alkylthio group, an aralkylthio group, an arylthio group, a heteroarylthio group, a halogen atom, an alkylenedioxy group, an amino group, a substituted amino group, a hydrazino group, a cyano group, a nitro group, a hydroxyl group, a carboxyl group, a hydroxamic acid group, a sulfonylamino group, a sulfamoyl group, a substituted sulfamoyl group, a carbamoyl group, a substituted carbamoyl group, a sulfo group, a sulfonyl group, a sulfino group, a sulfinyl group, an ureido group, a substituted ureido group, a mercapto group, a phosphoramide group, a substituted silyl group, a polymerizable group, or the like.

Examples of the hydrocarbon group may include an alkyl group, an alkenyl group, an alkynyl group, an aryl group, and an aralkyl group.

The alkyl group may be straight-chained, branched, or cyclic. For example, mention may be made of an alkyl group having 1 to 15 carbon atoms, preferably 1 to 10 carbon atoms, and more preferably 1 to 6 carbon atoms. Specifically, mention may be made of a methyl group, an ethyl group, an n-propyl group, a 2-propyl group, an n-butyl group, a 2-butyl group, an isobutyl group, a tert-butyl group, an n-pentyl group, a 2-pentyl group, a tert-pentyl group, a 2-methylbutyl group, a 3-methylbutyl group, a 2,2-dimethylpropyl group, an n-hexyl group, a 2-hexyl group, a 3-hexyl group, a 2-methylpentyl group, a 3-methylpentyl group, a 4-methylpentyl group, a 2-methylpentan-3-yl group, a cyclopropyl group, a cyclobutyl group, a cyclopentyl group, a cyclohexyl group, or the like.

The alkenyl group may be straight-chained or branched. For example, mention may be made of an alkenyl group having 2 to 15 carbon atoms, preferably 2 to 10 carbon atoms, and more preferably 2 to 6 carbon atoms. Specifically, mention may be made of an ethenyl group, a propenyl group, a 1-butenyl group, a pentenyl group, a hexenyl group, or the like.

The alkynyl group may be straight-chained or branched. For example, mention may be made of an alkynyl group having 2 to 15 carbon atoms, preferably 2 to 10 carbon atoms, and more preferably 2 to 6 carbon atoms. Specifically, mention may be made of an ethynyl group, a 1-propynyl group, a 2-propynyl group, 1-butynyl group, a 3-butynyl group, a pentynyl group, a hexynyl group, or the like.

As the aryl group, for example, mention may be made of an aryl group having 6 to 14 carbon atoms. Specifically, mention may be made of a phenyl group, a naphthyl group, an anthryl group, a biphenyl group, or the like.

As the aralkyl group, mention may be made of a group obtained by substituting at least one hydrogen atom of the alkyl group with the aryl group. For example, an aralkyl group having 7 to 12 carbon atoms is preferred. Specifically, mention may be made of a benzyl group, a 2-phenethyl group, a 1-phenylpropyl group, a 3-naphthylpropyl group, or the like.

As the aliphatic heterocyclic group, for example, mention may be made of a 5- to 8-membered, preferably 5- or 6-membered monocyclic aliphatic heterocyclic group, or polycyclic or fused ring aliphatic heterocyclic group, containing 2 to 14 carbon atoms, and at least one, preferably 1 to 3 hetero atoms such as nitrogen atoms, oxygen atoms, or sulfur atoms as hetero atoms. Specific examples of the aliphatic heterocyclic group may include a pyrrolidyl-2-one group, a piperidino group, a piperadinyl group, a morpholino group, a tetrahydrofuryl group, and a tetrahyropyranyl group.

As the aromatic heterocyclic group, for example, mention may be made of a 5- to 8-membered, preferably 5- or 6-membered monocyclic heteroaryl group, or polycyclic or fused ring heteroaryl group, containing 2 to 15 carbon atoms, and at least one, preferably 1 to 3 hetero atoms such as nitrogen atoms, oxygen atoms, or sulfur atoms as hetero atoms. Specifically, mention may be made of a furyl group, a thienyl group, a pyridyl group, a pyrimidyl group, a pyrazyl group, a pyridazyl group, a pyrazolyl group, an imidazolyl group, an oxazolyl group, a thiazolyl group, a benzofuryl group, a benzothienyl group, a quinolyl group, an isoquinolyl group, a quinoxalyl group, a phthalazyl group, a quinazolyl group, a naphthyridyl group, a cinnolyl group, a benzimidazolyl group, a benzoxazolyl group, a benzothiazolyl group, or the like.

The alkoxy group may be straight-chained, branched, or cyclic. For example, mention may be made of an alkoxy group having 1 to 6 carbon atoms. Specifically, mention may be made of a methoxy group, an ethoxy group, an n-propoxy group, a 2-propoxy group, an n-butoxy group, a 2-butoxy group, an isobutoxy group, a tert-butoxy group, an n-pentyloxy group, a 2-methylbutoxy group, a 3-methylbutoxy group, a 2,2-dimethylpropyloxy group, an n-hexyloxy group, a 2-methylpentyloxy group, a 3-methylpentyloxy group, a 4-methylpentyloxy group, a 5-methylpentyloxy group, a cyclohexyloxy group, or the like.

As the aryloxy group, for example, mention may be made of an aryloxy group having 6 to 14 carbon atoms. Specifically, mention may be made of a phenyloxy group, a naphthyloxy group, an anthryloxy group, or the like.

As the aralkyloxy group, for example, mention may be made of an aralkyloxy group having 7 to 12 carbon atoms. Specifically, mention may be made of a benzyloxy group, a 2-phenetyloxy group, a 1-phenylpropoxy group, a 2-phenylpropoxy group, a 3-phenylpropoxy group, a 1-phenylbutoxy group, a 2-phenylbutoxy group, a 3-phenylbutoxy group, a 4-phenylbutoxy group, a 1-phenylpentyloxy group, a 2-phenylpentyloxy group, a 3-phenylpentyloxy group, a 4-phenylpentyloxy group, a 5-phenylpentyloxy group, a 1-phenylhexyloxy group, a 2-phenylhexyloxy group, a 3-phenylhexyloxy group, a 4-phenylhexyloxy group, a 5-phenylhexyloxy group, a 6-phenylhexyloxy group, or the like.

As the heteroaryloxy group, for example, mention may be made of a heteroaryloxy group containing 2 to 14 carbon atoms, and at least one, preferably 1 to 3 hetero atoms such as nitrogen atoms, oxygen atoms, or sulfur atoms as hetero atoms. Specifically, mention may be made of a 2-pyridyloxy group, a 2-pyrazyloxy group, a 2-pyrimidyloxy group, a 2-quinolyloxy group, or the like.

The alkoxycarbonyl group may be straight-chained, branched, or cyclic. For example, mention may be made of an alkoxycarbonyl group having 2 to 19 carbon atoms. Specifically, mention may be made of a methoxycarbonyl group, an ethoxycarbonyl group, an n-propoxycarbonyl group, a 2-propoxycarbonyl group, an n-butoxycarbonyl group, a tert-butoxycarbonyl group, a pentyloxycarbonyl group, a hexyloxycarbonyl group, a 2-ethylhexyloxycarbonyl group, a lauryloxycarbonyl group, a stearyloxycarbonyl group, a cyclohexyloxycarbonyl group, or the like.

As the aryloxycarbonyl group, for example, mention may be made of an aryloxycarbonyl group having 7 to 20 carbon atoms. Specifically, mention may be made of a phenoxycarbonyl group, a naphthyloxycarbonyl group, or the like.

As the aralkyloxycarbonyl group, for example, mention may be made of an aralkyloxy group having 8 to 15 carbon atoms. Specifically, mention may be made of a benzyloxycarbonyl group, a phenethyloxycarbonyl group, a 9-fluorenyloxycarbonyl group, or the like.

The acyl group may be straight-chained or branched. For example, mention may be made of an acyl group having 1 to 18 carbon atoms derived from a carboxylic acid such as an aliphatic carboxylic acid or an aromatic carboxylic acid. Specifically, mention may be made of a formyl group, an acetyl group, a propionyl group, a butyryl group, a pivaloyl group, a pentanoyl group, a hexanoyl group, a lauroyl group, a stearoyl group, a benzoyl group, or the like.

As the acyloxy group, for example, mention may be made of an acyloxy group having 2 to 18 carbon atoms, derived from a carboxylic acid. Specifically, mention may be made of an acetoxy group, a propionyloxy group, a butyryloxy group, a pivaloyloxy group, a pentanoyloxy group, a hexanoyloxy group, a lauroyloxy group, a stearolyoxy group, a benzoyloxy group, or the like.

The alkylthio group may be straight-chained, branched, or cyclic. For example, mention may be made of an alkylthio group having 1 to 6 carbon atoms. Specifically, mention may be made of a methylthio group, an ethylthio group, an n-propylthio group, a 2-propylthio group, an n-butylthio group, a 2-butylthio group, an isobutylthio group, a tert-butylthio group, a pentylthio group, a hexylthio group, a cyclohexylthio group, or the like.

As the arylthio group, for example, mention may be made of an arylthio group having 6 to 14 carbon atoms. Specifically, mention may be made of a phenylthio group, a naphthylthio group, or the like.

As the aralkylthio group, for example, mention may be made of an aralkylthio group having 7 to 12 carbon atoms. Specifically, mention may be made of a benzylthio group, a 2-phenethylthio group, or the like.

As the heteroarylthio group, for example, mention may be made of a heteroarylthio group containing 2 to 14 carbon atoms, and at least one, preferably 1 to 3 hetero atoms such as nitrogen atoms, oxygen atoms, or sulfur atoms as hetero atoms. Specifically, for example, mention may be made of a 4-pyridylthio group, a 2-benzimidazolylthio group, a 2-benzoxazolylthio group, or a 2-benzothiazolylthio group.

As the halogen atom, mention may be made of a fluorine atom, a chlorine atom, a bromine atom, an iodine atom, or the like.

As the alkylenedioxy group, for example, mention may be made of an alkylenedioxy group having 1 to 3 carbon atoms. Specifically, mention may be made of a methylenedioxy group, an ethylenedioxy group, a propylenedioxy group, or the like.

As the substituted hydrocarbon group, for example, mention may be made of a substituted alkyl group, a substituted alkenyl group, a substituted alkynyl group, a substituted aryl group, or a substituted aralkyl group.

As the substituted alkyl group, mention may be made of an alkyl group obtained by substituting at least one hydrogen atom of the alkyl group with a substituent such as an alkyl group, an alkoxy group, a halogen atom, an amino group, or a substituted amino group. The alkyl group, the alkoxy group, and the halogen atom are the same as described above. Whereas, the substituted amino group is the same as the substituted amino group described later. As the alkyl group substituted with a halogen atom, i.e., the haloalkyl group, mention may be made of a haloalkyl group having 1 to 15 carbon atoms, preferably 1 to 10 carbon atoms, and more preferably 1 to 6 carbon atoms, obtained by halogenating (e.g., fluorinating, chlorinating, brominating, or iodinating) at least one hydrogen atom of the alkyl group with a halogen atom. Specifically, mention may be made of a chloromethyl group, a bromomethyl group, a trifluoromethyl group, a 2-chloroethyl group, a 3-bromomethyl group, a 3,3,3-trifluoropropyl group, or the like.

As the substituted aryl group, mention may be made of an aryl group obtained by substituting at least one hydrogen atom of the aryl group with a substituent such as an alkyl group, a haloalkyl group, an alkoxy group, a halogen atom, an amino group, or a substituted amino group, or an aryl group obtained by substituting the adjacent two hydrogen atoms of the aryl group with substituents such as alkylenedioxy groups. The alkyl group, the haloalkyl group, the alkoxy group, the halogen atom, the substituted amino group, and the alkylenedioxy group are the same as described above. Whereas, the substituted amino group is the same as the substituted amino group described later. Specific examples of the aryl group substituted with an alkyl group may include a tolyl group and a xylyl group.

As the substituted aralkyl group, mention may be made of an aralkyl group obtained by substituting at least one hydrogen atom of the aralkyl group with a substituent such as an alkyl group, a haloalkyl group, an alkoxy group, a halogen atom, an amino group, or a substituted amino group, or an aralkyl group obtained by substituting the adjacent two hydrogen atoms of the aryl group in the aralkyl group with substituents such as alkylenedioxy groups. The alkyl group, the haloalkyl group, the alkoxy group, the halogen atom, and the substituted amino group are the same as described above. Whereas, the substituted amino group is the same as the substituted amino group described later.

As the substituted aliphatic heterocyclic group, mention may be made of an aliphatic heterocyclic group obtained by substituting at least one hydrogen atom of the aliphatic heterocyclic group with a substituent such as an alkyl group, a haloalkyl group, an alkoxy group, or a halogen atom. The alkyl group, the haloalkyl group, the alkoxy group, and the halogen atom are the same as described above.

As the substituted aromatic heterocyclic group, mention may be made of a heteroaryl group obtained by substituting at least one hydrogen atom of the heteroaryl group with a substituent such as an alkyl group, a haloalkyl group, an alkoxy group, or a halogen atom. The alkyl group, the haloalkyl group, the alkoxy group, and the halogen atom are the same as described above.

As the substituted alkoxy group, mention may be made of an alkoxy group obtained by substituting at least one hydrogen atom of the alkoxy group with a substituent such as an alkyl group, a haloalkyl group, an alkoxy group, a halogen atom, an amino group, or a substituted amino group. The alkyl group, the haloalkyl group, the alkoxy group, and the halogen atom are the same as described above. Whereas, the substituted amino group is the same as the substituted amino group described later.

As the substituted aryloxy group, mention may be made of an aryloxy group obtained by substituting at least one hydrogen atom of the aryloxy group with a substituent such as an alkyl group, a haloalkyl group, an alkoxy group, a halogen atom, an amino group, or a substituted amino group, an aryloxy group obtained by substituting the adjacent two hydrogen atoms of the aryloxy group with an alkylenedioxy group or the like. The alkyl group, the haloalkyl group, the alkoxy group, the halogen atom, and the alkylenedioxy group are the same as described above. Whereas, the substituted amino group is the same as the substituted amino group described later.

As the substituted aralkyloxy group, mention may be made of an aralkyloxy group obtained by substituting at least one hydrogen atom of the aralkyloxy group with a substituent such as an alkyl group, a haloalkyl group, an alkoxy group, a halogen atom, an amino group, or a substituted amino group, an aralkyloxy group obtained by substituting the adjacent two hydrogen atoms of the aryl group in the aralkyloxy group with alkylenedioxy groups or the like. The alkyl group, the haloalkyl group, the alkoxy group, the halogen atom, and the alkylenedioxy group are the same as described above. Whereas, the substituted amino group is the same as the substituted amino group described later.

As the substituted amino group, mention may be made of an amino group obtained by substituting one or two hydrogen atoms of the amino group with a substituent such as a protective group. Any protective groups can be used so long as they are used as amino protective groups. For example, mention may be made of the ones described as amino protective groups in PROTECTIVE GROUPS IN ORGANIC SYNTHESIS Second Edition (JOHN WILEY & SONS, INC.). Specific examples of the amino protective group may include an alkyl group, an aryl group, an aralkyl group, an acyl group, an alkoxycarbonyl group, an aryloxycarbonyl group, and an aralkyloxycarbonyl group.

The alkyl group, the aryl group, the aralkyl group, the acyl group, the alkoxycarbonyl group, the aryloxycarbonyl group, and the aralkyloxycarbonyl group are the same as described above. Specific examples of the amino group substituted with an alkyl group, i.e., an alkyl-substituted amino group may include mono- or di-alkylamino groups such as an N-methylamino group, an N,N-dimethylamino group, an N,N-diethylamino group, an N,N-diisopropylamino group, and an N-cyclohexylamino group. Specific examples of the amino group substituted with an aryl group, i.e., an aryl-substituted amino group may include mono- or di-arylamino groups such as an N-phenylamino group, an N,N-diphenylamino group, an N-naphthylamino group, and an N-naphthyl-N-phenylamino group. Specific examples of the amino group substituted with an aralkyl group, i.e., an aralkyl-substituted amino group may include mono- or di-aralkylamino groups such as an N-benzylamino group and an N,N-dibenzylamino group. Specific examples of the amino group substituted with an acyl group, i.e., an acylamino group may include a formylamino group, an acetylamino group, a propionylamino group, a pivaloylamino group, a pentanoylamino group, a hexanoylamino group, and a benzoylamino group. Specific examples of the amino group substituted with an alkoxycarbonyl group, i.e., an alkoxycarbonylamino group may include a methoxycarbonylamino group, an ethoxycarbonylamino group, an n-propoxycarbonylamino group, an n-butoxycarbonylamino group, a tert-butoxycarbonylamino group, a pentyloxycarbonylamino group, and a hexyloxycarbonylamino group. Specific examples of the amino group substituted with an aryloxycarbonyl group, i. e. , the aryloxycarbonylamino group may include an amino group obtained by substituting one hydrogen atom of the amino group with the aryloxycarbonyl group. Specifically, mention may be made of a phenoxycarbonylamino group, a naphthyloxycarbonyl amino group, and the like. Specific examples of the amino group substituted with an aralkyloxycarbonyl group, i.e., the aralkyloxycarbonylamino group may include a benzyloxycarbonylamino group.

As the sulfonylamino group, mention may be made of, for example, a substituted sulfonylamino group represented by R-SO₂-NH- (where, R represents an alkyl group, a substituted alkyl group, an aryl group, a substituted aryl group, an aralkyl group, a substituted aralkyl group, or the like). The alkyl group, the substituted alkyl group, the aryl group, the substituted aryl group, the aralkyl group, and the substituted aralkyl group represented by R are the same as described above. Specific examples of the sulfonylamino group may include a methanesulnonylamino group and a p-toluenesulfonylamino group.

As the substituted sulfamoyl group, mention may be made of a sulfamoyl group obtained by substituting one or two hydrogen atoms of the amino group in the sulfamoyl group with a substituent such as the alkyl group, the substituted alkyl group, the aryl group, the substituted aryl group, the aralkyl group, or the substituted aralkyl group. Specifically, mention may be made of an N-methylsulfamoyl group, an N,N-dimethylsulfamoyl group, an N-phenylsulfamoyl group, or the like.

As the substituted carbamoyl group, mention may be made of a carbamoyl group obtained by substituting one or two hydrogen atoms of the amino group in the carbamoyl group with a substituent such as the alkyl group, the substituted alkyl group, the aryl group, the substituted aryl group, the aralkyl group, or the substituted aralkyl group. Specifically, mention may be made of an N-methylcarbamoyl group, an N,N-diethylcarbamoyl group, an N-phenylcarbamoyl group, or the like.

As the sulfonyl group, for example, mention may be made of a substituted sulfonyl group represented by R-SO₂- (where R is the same as described above). Specific examples of the sulfonyl group may include a methanesulfonyl group and a p-toluenesulfonyl group.

As the sulfinyl group, for example, mention may be made of a substituted sulfinyl group represented by R-SO- (where R is the same as described above). Specific examples of the sulfinyl group may include a methanesulfinyl group and a benzenesulfinyl group.

As the substituted ureido group, mention may be made of an ureido group obtained by substituting one or two hydrogen atoms of the amino group in the ureido group and/or one hydrogen of the imino group in the ureido group with a substituent such as the alkyl group, the substituted alkyl group, the aryl group, the substituted aryl group, the aralkyl group, or the substituted aralkyl group. Specifically, mention may be made of an N-methylureido group, an N-phenylureido group, or the like.

As the phosphoramide group, mention may be made of a substituted phosphoramide group obtained by substituting at least one hydrogen atom of the phosphoric acid group in the phosphoramide group with a substituent such as the alkyl group, the substituted alkyl group, the aryl group, the substituted aryl group, the aralkyl group, or the substituted aralkyl group. Specifically, mention may be made of a diethylphosphoramide group, a phenylphosphoramide group, or the like.

As the substituted silyl group, mention may be made of a tri-substituted silyl group obtained by substituting three hydrogen atoms of the silyl group each with a substituent such as the alkyl group, the substituted alkyl group, the aryl group, the substituted aryl group, the aralkyl group, or the substituted aralkyl group. Specifically, mention may be made of a trimethylsilyl group, a tert-butyldimethylsilyl group, a tert-butyldiphenylsilyl group, a triphenylsilyl group, or the like.

As the polymerizable group, mention may be made of, for example, a group having a polymerizable double bond or triple bond such as a vinyl group, a propenyl group, an ethynyl group, or a (meth)acryloyloxy group, or for example, a cyclic ether group capable of ring-opening polymerization such as an oxiranyl group, a tetrahydrofuryl group, or a tetrahyropyranyl group.

Out of these substituents, the substituted hydrocarbon group, the substituted aliphatic heterocyclic group, the substituted aromatic heterocyclic group, the substituted alkoxy group, the substituted aryloxy group, the substituted aralkyloxy group, the substituted heteroaryloxy group, the alkoxycarbonyl group, the aryloxycarbonyl group, the aralkyloxycarbonyl group, the acyl group, the acyloxy group, the alkylthio group, the aralkylthio group, the arylthio group, the heteroarylthio group, the alkylenedioxy group, the substituted amino group, the hydrazino group, the hydroxamic acid group, the substituted sulfamoyl group, the substituted carbamoyl group, the sulfonyl group, the sulfinyl group, the substituted ureido group, the phosphoramide group, or the substituted silyl group may be further substituted with a group selected from the group of the substituents.

As each aryl group which may be substituted, represented by the ring A, the ring B, and the ring C in the general formula [1], the aryl group which may be substituted, represented by the ring C₁ in the general formula [1'], and the aryl group which may be substituted, represented by the ring A₂, in the general formulae [1''], [1a''], [1'''], and [1a"'], mention may be made of an aryl group, and a substituted aryl group. Whereas, as the heteroaryl group which may be substituted, mention may be made of a heteroaryl group and a substituted heteroaryl group.

The aryl group may be a monocyclic, polycyclic, or fused ring aryl group. For example, mention may be made of an aryl group having 6 to 14 carbon atoms. Specifically, mention may be made of a phenyl group, a naphthyl group, an anthryl group, a biphenyl group, or the like.

As the substituted aryl group, mention may be made of an aryl group obtained by substituting at least one hydrogen atom of the aryl group with a substituent. As the substituents, mention may be made of the same groups as the substituents described in details previously in connection with the substituents in the substituted nitrogen-containing aromatic heterocyclic group.

As the heteroaryl group, for example, mention may be made of a 5- to 8-membered, preferably 5- or 6-membered monocyclic heteroaryl group, or polycyclic or fused ring heteroaryl group, containing 2 to 15 carbon atoms, and at least one, preferably 1 to 3 hetero atoms such as nitrogen atoms, oxygen atoms, or sulfur atoms as hetero atoms. Specifically, mention may be made of a furyl group, a thienyl group, a pyridyl group, a pyrimidyl group, a pyrazyl group, a pyridazyl group, a pyrazolyl group, an imidazolyl group, an oxazolyl group, a thiazolyl group, a benzofuryl group, a benzothienyl group, a quinolyl group, an isoquinolyl group, a quinoxalyl group, a phthalazyl group, a quinazolyl group, a naphthyridyl group, a cinnolyl group, a benzimidazolyl group, a benzoxazolyl group, a benzothiazolyl group, or the like.

As the substituted heteroaryl group, mention may be made of a substituted heteroaryl group obtained by substituting at least one hydrogen atom of the heteroaryl group with a substituent. As the substituents, mention may be made of the same groups as the substituents described in details previously in connection with the substituents in the substituted nitrogen-containing aromatic heterocyclic group.

Whereas, specific examples of the fused ring when the ring C₂ and the ring B₂ are bonded to each other to form a fused ring may include a 1,10-phenanthroline ring and a 4,5-diazafluoren-9-one ring. Specific examples of the fused ring when the ring A₁ and the ring C₁ (or the ring C₁ and the ring B₁), or the ring A₂ and the ring C₂ are bonded to each other to form a fused ring may include a 1,7-phenanthroline ring and a 7,8-benzoquinoline ring.

Incidentally, the respective three rings of the ring A, the ring C, and the ring B; the ring A₁, the ring C₁, and the ring B₁; or the ring B₂, the ring C₂, and the ring A₂ may be mutually bonded to form a fused ring.

In the general formula [1], as the halogen atom represented by Y, for example, mention may be made of a chlorine atom, a bromine atom, an iodine atom, or a fluorine atom.

Whereas, as the aryl group which may be substituted and the heteroaryl group which may be substituted when Y is an aryl group which may be substituted or a heteroaryl group which may be substituted, bonded directly, or through an oxygen atom (-O-) or a sulfur atom (-S-), mention may be made of the same ones as the aryl group which may be substituted and the heteroaryl group which may be substituted, represented by the ring A.

In the general formulae [1'], [1a'], and [1b'], as the halogen atom represented by X, for example, mention may be made of a chlorine atom, a bromine atom, an iodine atom, or a fluorine atom.

In the general formulae [1'''] and [1a"'], as the aryl group which may be substituted represented by E and the heteroaryl group which may be substituted, mention may be made of the same ones as the aryl group which may be substituted and the heteroaryl group which may be substituted, represented by the ring A.

In the general formulae [1a'], [1b'], [1a''], [1a'''], and [1b'''], the definitions and the specific examples of various substituents represented by R¹, R², and R³ are entirely the same as the ones previously described for the explanation of the substituents of the ring B and the ring C of the general formulae [1], [1'], [1"], and [1"'].

Preferred specific examples of the platinum complex of the invention represented by the general formula [1'] may include the platinum complexes represented by the following (1'-1) to (1'-32):

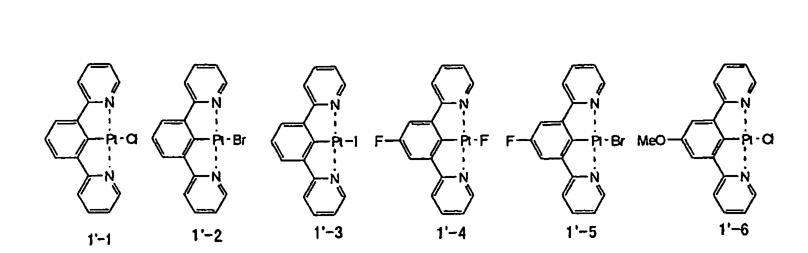

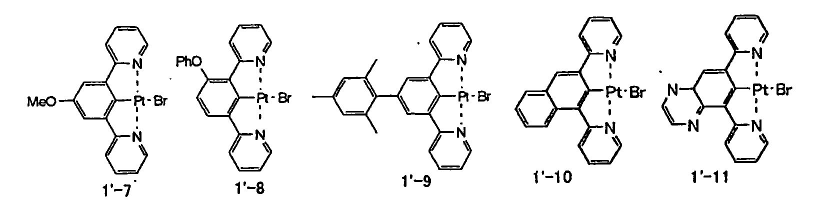

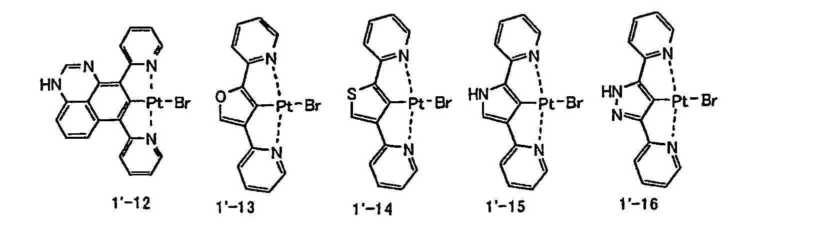

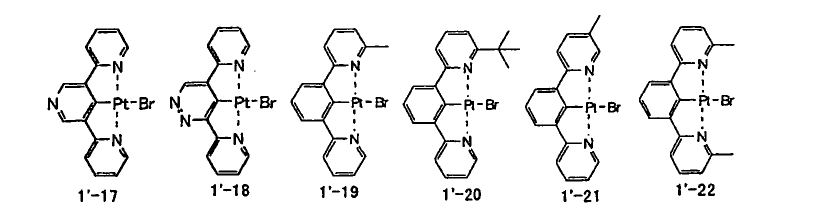

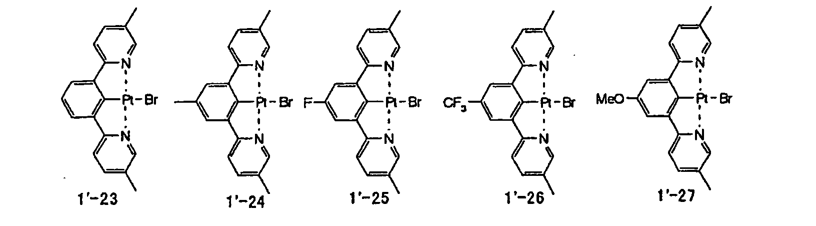

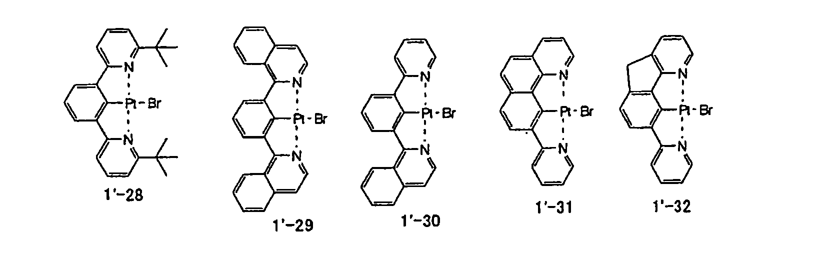

Preferred specific examples of the platinum complex of the invention represented by the general formula [1"] may include the platinum complexes represented by the following (1"-1) to (1''-21):

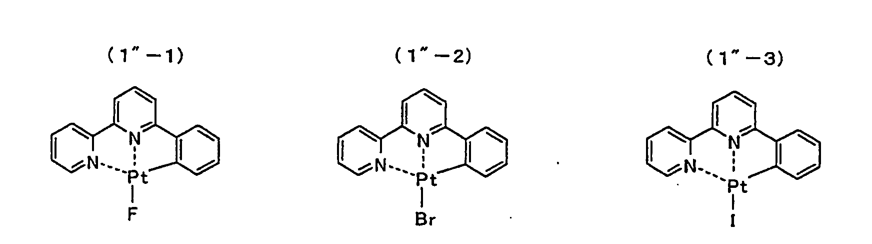

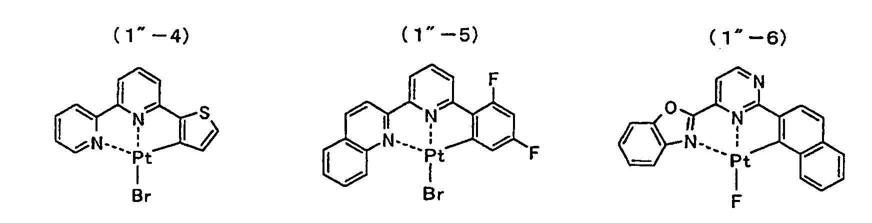

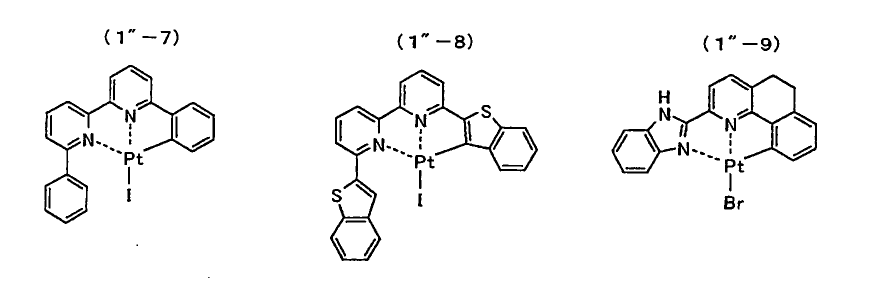

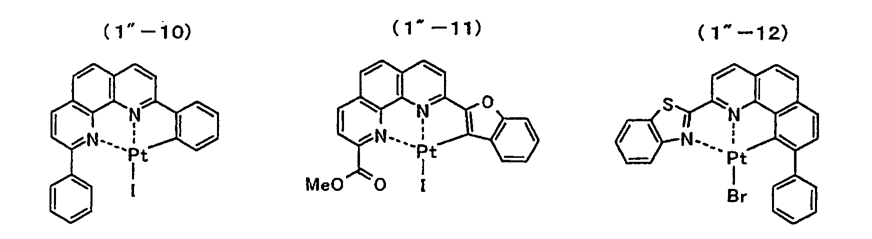

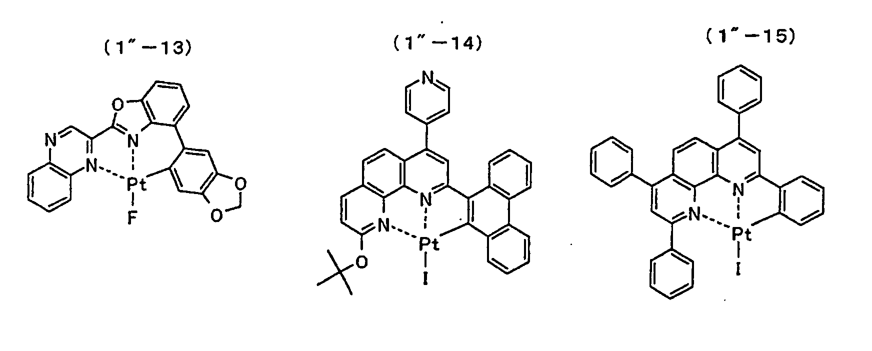

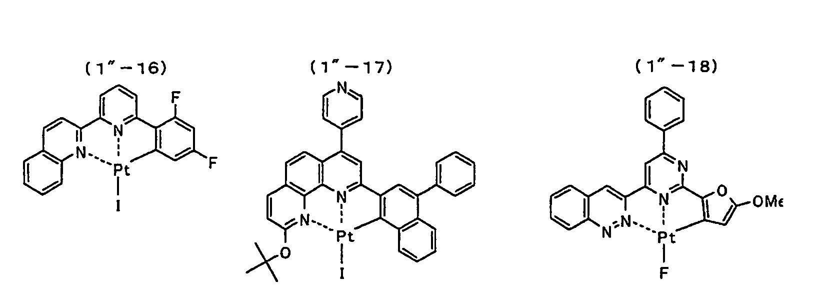

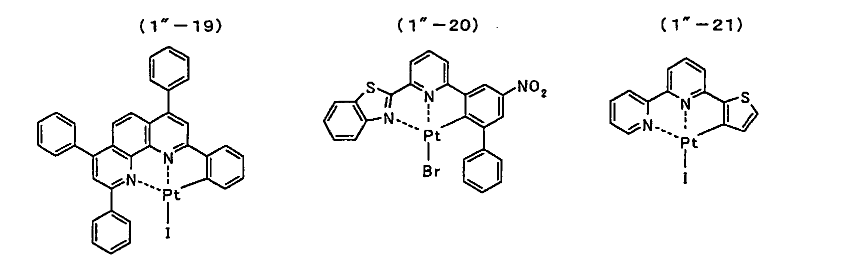

Preferred specific examples of the platinum complex of the invention represented by the general formula [1'''] may include the platinum complexes represented by the following (1"'-1) to (1"'-15) :

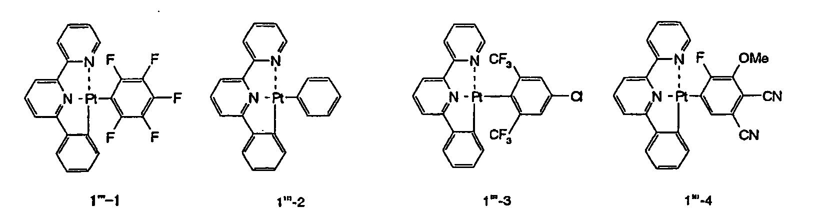

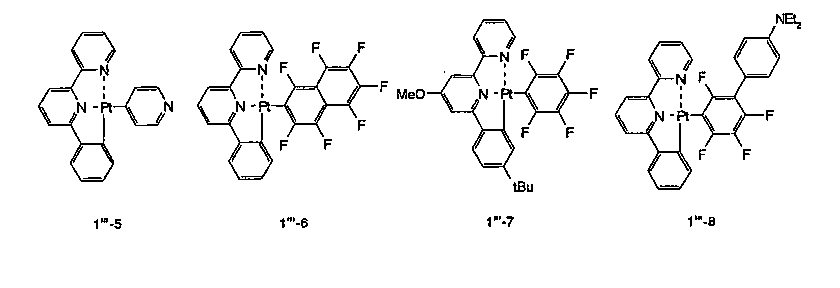

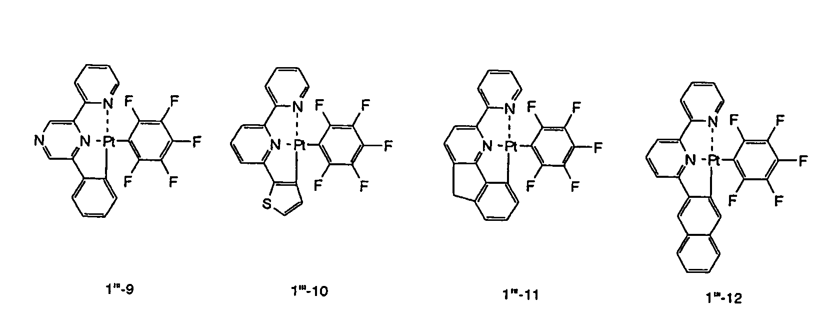

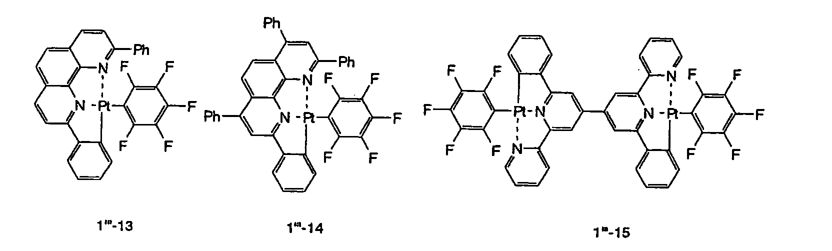

The platinum complexes of the invention represented by the general formula [1'] can be synthesized by, for example, the following method. Namely, as described in Organometallics 1999, 18, p3337 to p3341, a platinum compound such as potassium tetrachloroplatinate and a ligand such as 1,3-di(2-pyridyl)benzene are heated with stirring in a solvent such as acetic acid. As the raw material platinum compound herein used, mention may be made of those such as potassium tetrachloroplatinate, potassium tetrabromoplatinate, and sodium tetrachloroplatinate. Alternatively, a hydrate thereof may also be used. Whereas, as the reaction solvent, acetic acid, 2-ethoxyethanol, acetonitrile, or the like can be used. The solvent may be mixed with water to serve as a hydrous solvent for effecting the reaction. Further, the reaction may be effected at a reaction temperature of 30 to 150 °C, and preferably 70 to 100 °C. The platinum complex obtained in this step is generally a hydrate. If it remains unchanged, purification such as sublimation is difficult to perform thereon, and the hydrate cannot be used as a light emitting device material because it contains water.

In order to remove the hydrated water, it is possible to remove the water hydrated with an organometallic compound such as a Grignard reagent. Further, in the step of removing the hydrated water, by allowing the halogen atom bonded to the platinum complex hydrate to react with a Grignard reagent containing another halogen atom at a temperature of about 0 to 80 °C, and preferably 10 to 40 °C, it is possible to perform removal of the hydrated water and exchange of the halogen atoms in one step. As the Grignard reagent herein used, both an alkyl Grignard reagent and an aryl Grignard reagent can be used. However, an aryl Grignard reagent is preferred, and particularly, a phenyl Grignard reagent is preferred.

The platinum complex of the invention represented by the general formula [1"] can be synthesized, for example, by allowing the platinum diene complex represented by the following general formula [2]:

Pt(X¹)₂(D) [2]

(where in the formula, D represents a nonconjugated diene compound, and X¹ represents a fluorine atom, a bromine atom, or an iodine atom.) to react with the compound represented by the general formula [3]: 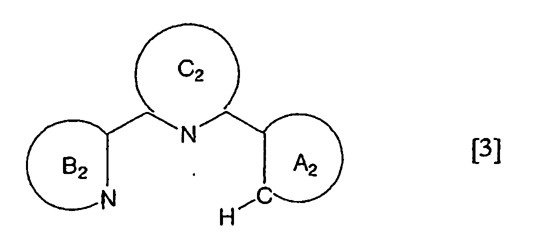 (where in the formula, the ring B₂ and the ring C₂ each independently represent a nitrogen-containing aromatic heterocyclic group which may be substituted, the ring A₂ represents an aryl group which may be substituted or a heteroaryl group which may be substituted, and the ring B₂ and the ring C₂, the ring C₂ and the ring A₂, or the ring B₂, the ring C₂, and the ring A₂ may be mutually bonded to form a fused ring to react with each other).

The platinum complex of the invention represented by the general formula [1''] can also be obtained, for example, by allowing the platinum diene complex represented by the following general formula [2a]:

Pt(Cl)₂(D) [2a]

(where in the formula, D represents a nonconjugated diene compound), the compound represented by the general formula [3] : 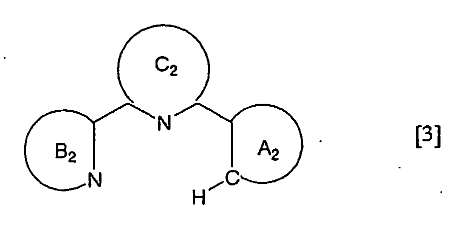 (where in the formula, the ring B₂, the ring C₂, and the ring A₂ are the same as described previously), and a halogenating agent for substituting a halogen atom other than chlorine to mutually react.

In this case, preferably, the platinum diene complex represented by the general formula [2a] is, first, allowed to react with the compound represented by the general formula [3], and then, allowed to react with a halogenating agent for substituting a halogen atom other than chlorine.

Further, the reaction of the platinum diene complex represented by the general formula [2a] with the compound represented by the general formula [3], and the subsequent reaction with the halogenating agent are more preferably effected in one pot.

The platinum complex of the invention represented by the general formula [1"] can also be obtained by allowing the platinum complex represented by the general formula [1b'']: 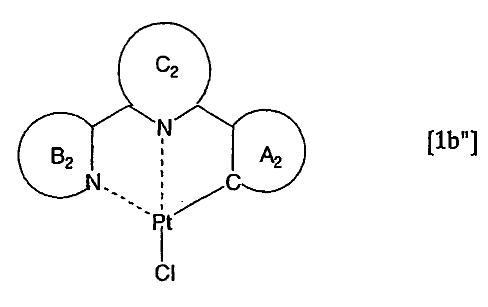 (where in the formula, the ring B₂, the ring C₂, and the ring A₂ are the same as described previously), and a halogenating agent for substituting a halogen atom other than chlorine to react with each other.

In the platinum diene complex of the invention represented by the general formula [2] or the general formula [2a] for use in the method for producing the platinum complex of the invention represented by the general formula [1"], the nonconjugated diene compound represented by D may be cyclic or noncyclic. When the nonconjugated diene compound is a cyclic nonconjugated diene compound, it may be any of a monocyclic, polycyclic, fused ring, and crosslinked ring ones. Alternatively, the nonconjugated diene compound may be a nonconjugated diene compound substituted with a substituent, i.e., a substituted nonconjugated diene compound. The substituent has no particular restriction so long as it is a substituent not adversely affecting the production method of the invention. As the substituents, mention may be made of the same groups as the substituents described in the explanation of the substituents of the substituted nitrogen-containing aromatic heterocyclic group. The nonconjugated diene compounds are especially preferably 1,5-cyclooctadiene, bicyclo[2,2,1]hepta-2,5-diene, 1, 5-hexadiene, and the like. As more preferred nonconjugated diene compound, mention may be made of 1,5-hexadiene, or the like.

In the method for producing the platinum complex represented by the general formula [1''], the compound represented by the general formula [3] to be allowed to react with the platinum diene complex represented by the general formula [2] is a compound having the ring B₂, the ring C₂, and the ring A₂, and a compound having functions of coordinating to a platinum atom through the ring B₂ and the ring C₂, and bonding to the platinum atom through a carbon atom on the ring A₂.

The definitions, the specific examples of the ring B₂, the ring C₂, and the ring A₂, and the like are as described previously.

Preferred examples of the compound represented by the general formula [3] may include the compounds represented by the following general formula [3a]: 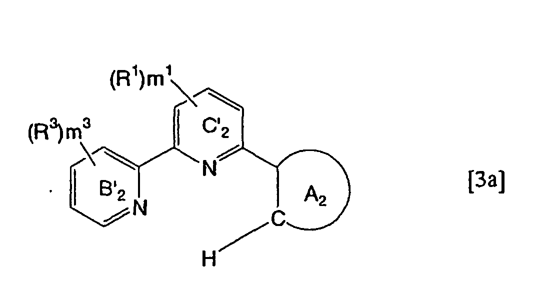 (where in the formula, the ring B'₂, the ring C'₂, the ring A₂, R¹, R³, m¹, and m³ are the same as described above).

Preferred specific examples of the compound represented by the general formula [3] may include those composed of the moiety obtained by removing platinum and halogen atoms from the structural formula of each preferred specific example of the platinum complexes (orthometalated platinum complexes each having a tridentate ligand and having a halogen atom) represented by the foregoing (1''-1) to (1"-21).

The platinum complexes represented by the general formula [1b''] for use in the method for producing the platinum complexes of the invention represented by the general formula [1"] are predominantly known compounds. They are generally produced by an ordinary method using potassium tetrachloroplatinate or the like. However, they can be produced more effectively by adopting the method for producing the platinum complex having a tridentate ligand, and having a halogen atom, in accordance with the invention, described later.

In the production method of the invention, as the halogenating agents to be used for substituting a halogen atom other than chlorine, for example, mention may be made of inorganic halogenating agents and organic halogenating agents such as metal halides, phosphorus halides, and halogens.

As the metal halides, for example, mention may be made of alkali metal halides such as lithium fluoride, lithium bromide, lithium iodide, sodium fluoride, sodium bromide, sodium iodide, potassium fluoride, potassium bromide, potassium iodide, cesium fluoride, cesium bromide, and cesium iodide, and alkaline earth metal halides such as magnesium fluoride, magnesium bromide, magnesium iodide, calcium bromide, and calcium iodide.

As the phosphorus halides, for example, mention may be made of phosphorus tribromide.

As the halogens, for example, mention may be made of halogens such as fluorine, bromine, and iodine.

As the organic halogenating agents, for example, mention may be made of succinimides such as N-bromosuccinimide.

Out of these halogenating agents, metal halides are particularly preferred.

The invention provides the platinum complexes of, for example, the general formula [1'], the general formula [1''], and the general formula [1"'], and the production method thereof. It also simultaneously provides the method for producing the platinum complex having a tridentate ligand, and having a halogen atom, characterized by using the platinum diene complex represented by the general formula [2b]:

Pt(X)₂(D) [2b]

(where in the formula, D represents a nonconjugated diene compound, and X represents a halogen atom.) as a platinum source.

Herein, as the platinum complex having a tridentate ligand, and having a halogen atom, for example, mention may be made of the platinum complex represented by the following general formula [1c'']: 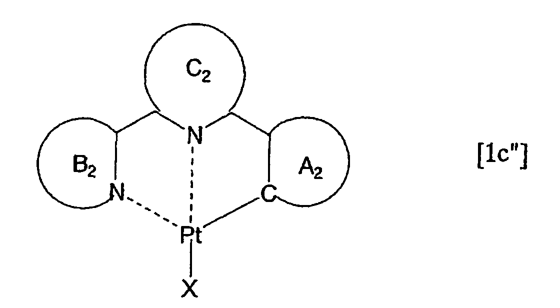 (where in the formula, the ring B₂, the ring C₂, and the ring A₂ are the same as described above, and X represents a halogen atom).

The production method of the invention using the platinum diene complex represented by the general formula [2b] as a platinum source is more specifically as follows: for example, the platinum diene complex represented by the general formula [2b]:

Pt(X)₂(D) [2b]

(where in the formula, D and X are the same as described above) and the compound represented by the general formula [3]: 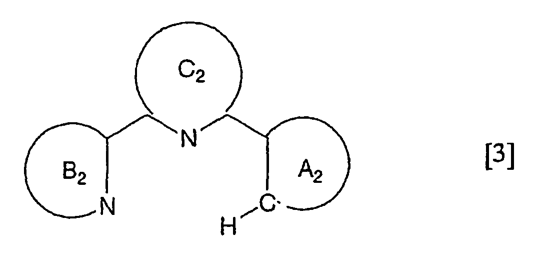 (where in the formula, the ring B₂, the ring C₂, and the ring A₂ are the same as described above) are allowed to react with each other, thereby to produce the platinum complex represented by the general formula [1c'']: 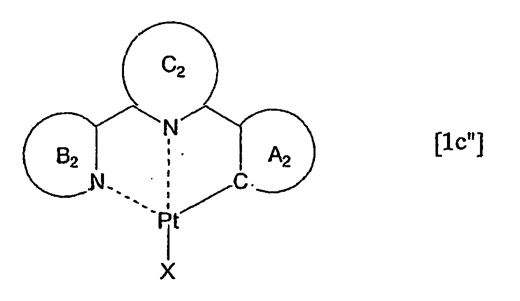 (where in the formula, the ring A₂, the ring B₂, and the ring C₂, and X are the same as described above).

Further, the production method of the invention is as follows: the platinum diene complex represented by the general formula [2b]:

Pt(X)₂(D) [2b]

(where in the formula, D and X are the same as described above), the compound represented by the general formula [3]: 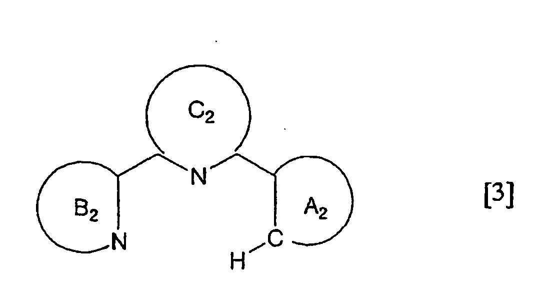 (where in the formula, the ring B₂, the ring C₂, and the ring A₂ are the same as described above), and a halogenating agent for substituting a halogen atom other than X are allowed to mutually react, thereby to produce the platinum complex represented by the general formula [1d'']: 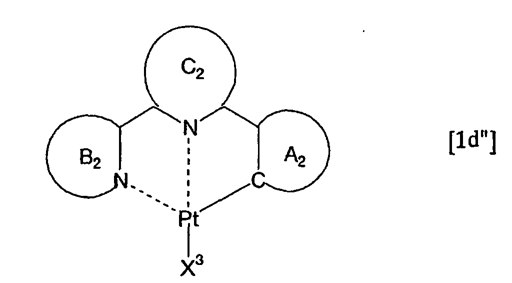 (where in the formula, the ring B₂, the ring C₂, and the ring A₂ are the same as described above, and X³ represents a halogen atom (provided that the cases where X and X³ are the same are excluded)).

In this case, preferably, the platinum diene complex represented by the general formula [2b] is, first, allowed to react with the compound represented by the general formula [3], and then, allowed to react with a halogenating agent for substituting a halogen atom other than X.

Further, the reaction of the platinum diene complex represented by the general formula [2b] with the compound represented by the general formula [3], and the subsequent reaction with the halogenating agent are more preferably effected in one pot.

The production method of the invention using the platinum diene complex represented by the general formula [2b] as a platinum source is also as follows: the platinum complex represented by the general formula [1c'']: 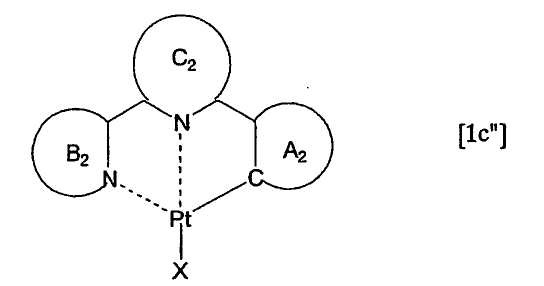 (where in the formula, the ring B₂, the ring C₂, the ring A₂, and X are the same as described above) and a halogenating agent for substituting a halogen atom other than X are allowed to mutually react, thereby to produce the platinum complex represented by the general formula [1d'']: 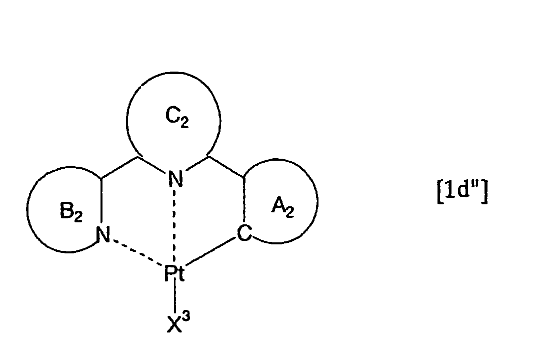 (where in the formula, the ring B₂, the ring C₂, the ring A₂, and X³ are the same as described above (provided that the cases where X and X³ are the same are excluded)).

As the halogenating agents for substituting a halogen atom other than X, for use in the production method of the invention, mention may be made of, other than the ones listed as the halogenating agents to be used for substituting a halogen atom other than chlorine, lithium chloride, sodium chloride, potassium chloride, cesium chloride, magnesium chloride, calcium chloride, phosphorus trichloride, chlorine, N-chlorosuccinimide, and the like.

In the general formula [1d''], as the halogen atom represented by X³, for example, mention may be made of a chlorine atom, a bromine atom, an iodine atom, or a fluorine atom. As a matter of course, when X is a chlorine atom, X³ is a halogen atom other than chlorine. When X is a bromine atom, X³ is a halogen atom other than bromine. As indicated by these cases and the like, it is needless to say that X ≠ X³.

Specific examples of the orthometalated platinum complex having a tridentate ligand, and having a halogen atom, obtained by the production method of the invention using the platinum diene complex represented by the general formula [2b] as a platinum source may include, other than the platinum complexes represented by (1''-1) to (1''-21) previously mentioned as the preferred specific examples of the platinum complex of the invention represented by the general formula [1''], the platinum complexes represented by (1b''-1) to (1b''-15) described below:

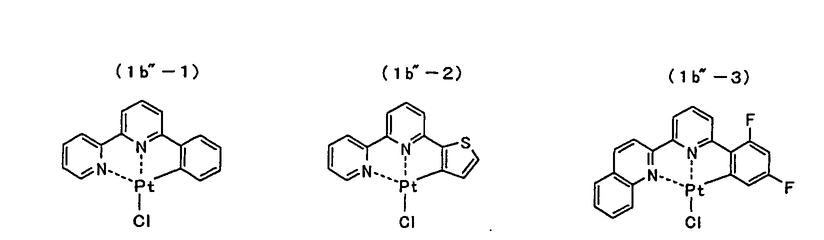

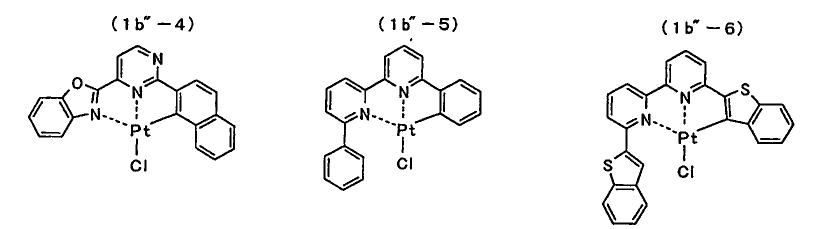

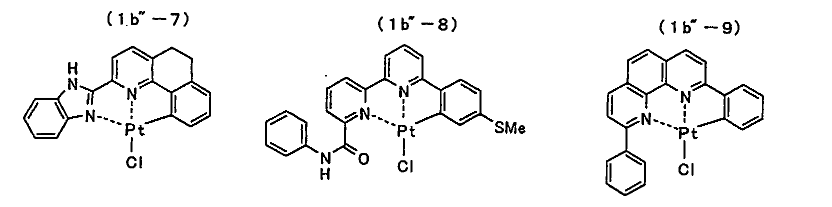

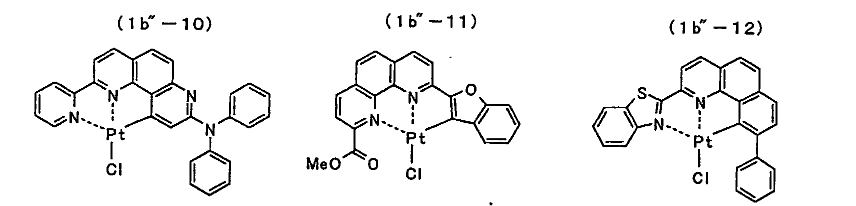

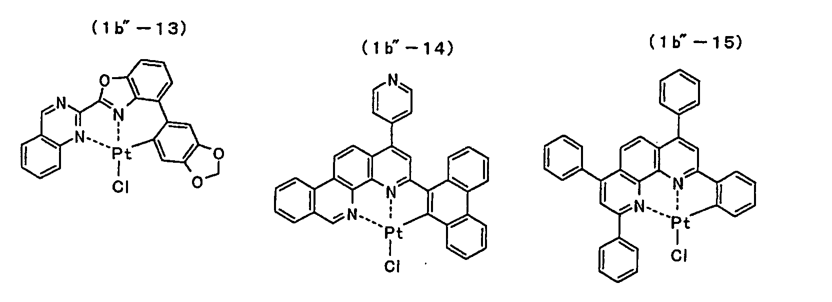

Then, the production method of the invention will be described in details by reference to the following reaction schemes 1 to 3:

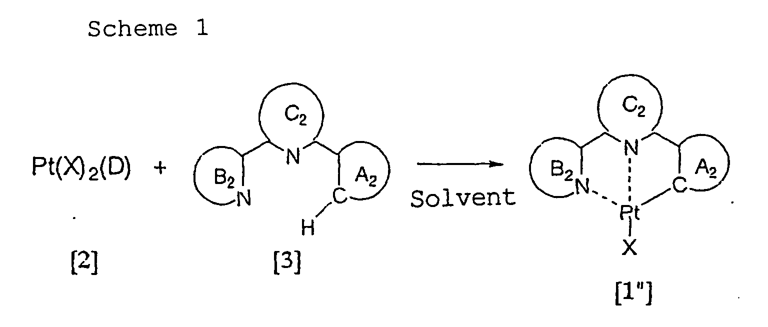

The scheme 1 is a formula for explaining the production method of the platinum complex represented by the general formula [1c''] (which is hereinafter abbreviated as a platinum complex [1c'']) using the platinum diene complex represented by the general formula [2b] (which is hereinafter abbreviated as a platinum diene complex [2b]) as a platinum source (which is hereinafter abbreviated as "the production method 1''").

The platinum complex [1c''] can be produced with ease by allowing the platinum diene complex [2b] and the compound represented by the general formula [3] (which is hereinafter abbreviated as the compound [3]) to react with each other in the presence of an appropriate solvent, if required, under an inert gas atmosphere.

Incidentally, the production method may also be accomplished by effecting the reaction, if required, using an ultrasonic generator in combination.

As for the amounts of the platinum diene complex [2b] and the compound [3], the amount of the compound [3] is appropriately selected from the range of generally 0.5 to 20 equivalents, and preferably 0.8 to 5 equivalents relative to the platinum diene complex [2b].

The production method 1" is preferably carried out in the presence of a solvent.

Examples of the solvent may include: amides such as N,N-dimethylformamide, formamide, and N,N-dimethylacetamide, nitriles such as acetonitrile, hydrocarbyl halides such as dichloromethane, 1,2-dichloroethane, chloroform, carbon tetrachloride, and o-dichlorobenzene, aliphatic hydrocarbons such as pentane, hexane, heptane, octane, decane, and cyclohexane, aromatic hydrocarbons such as benzene, toluene, and xylene, ethers such as diethyl ether, diisopropyl ether, tert-butyl methyl ether, dimethoxyethane, ethylene glycol diethyl ether, tetrahydrofuran, 1,4-dioxane, and 1,3-dioxolane, ketones such as acetone, methyl ethyl ketone, methyl isobutyl ketone, and cyclohexanone, alcohols such as methanol, ethanol, 2-propanol, n-butanol, and 2-ethoxyethanol, polyols such as ethylene glycol, propylene glycol, 1,2-propanediol, and glycerin, esters such as methyl acetate, ethyl acetate, n-butyl acetate, and methyl propionate, sulfoxides such as dimethyl sulfoxide, and water. These solvents may be respectively used alone, or in appropriate combination of two or more thereof. As the preferred solvents, mention may be made of ethers such as ethylene glycol diethyl ether, tetrahydrofuran, 1,4-dioxane, and 1,3-dioxolane, ketones such as acetone, methyl ethyl ketone, methyl isobutyl ketone, and cyclohexanone, alcohols such as methanol, ethanol, 2-propanol, n-butanol, and 2-ethoxyethanol, polyols such as ethylene glycol, propylene glycol, 1,2-propanediol, and glycerin, water, and the like. These preferred solvents may be respectively used alone, or in appropriate combination of two or more thereof.

The amount of the solvent to be used has no particular restriction so long as it is such an amount as to allow the reaction to proceed sufficiently. However, it is appropriately selected from the range of generally 1- to 200-fold volume, and preferably 1- to 50-fold volume relative the platinum diene complex [2b].

The production method 1" is preferably carried out under an inert gas atmosphere. As the inert gas, mention may be made of a nitrogen gas, an argon gas, or the like.

The reaction temperature is appropriately selected from the range of generally 25 to 300 °C, preferably 60 to 200 °C, and more preferably 80 to 150 °C.

Although the reaction time naturally varies depending upon the reaction temperature and other reaction conditions, it is appropriately selected from the range of generally 10 minutes to 72 hours, preferably 30 minutes to 48 hours, and more preferably 1 to 12 hours.

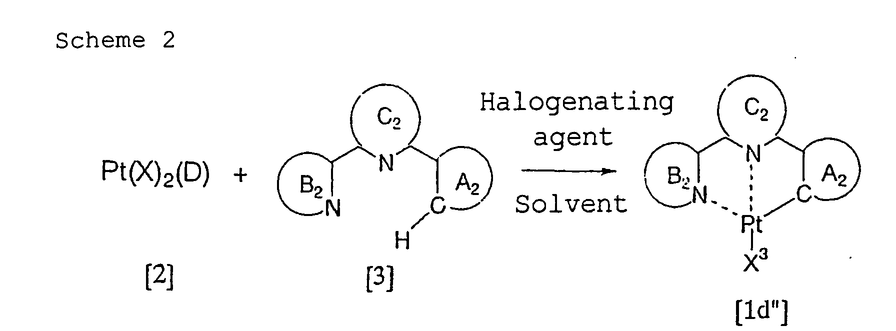

The scheme 2 is a formula for explaining the production method of the platinum complex having a desirable halogen atom [1c''] (which is hereinafter abbreviated as a platinum complex [1d"]) by allowing the platinum diene complex [2b], the compound [3], and a halogenating agent for substituting a desirable halogen atom to mutually react (which is hereinafter abbreviated as the "production method 2''").

The platinum complex [1d''] can be produced with ease by mixing the platinum diene complex [2b], the compound [3], and a halogenating agent for substituting a desirable halogen atom, and allowing them to mutually react in the presence of an appropriate solvent, if required, under an inert gas atmosphere.

Alternatively, the platinum complex [1d"] can also be obtained with ease in the following manner. First, the platinum diene complex [2b] and the compound [3] are allowed to react with each other in the presence of an appropriate solvent, if required, under an inert gas atmosphere. After the completion of the reaction, a post treatment and the like are carried out to isolate the product. Then, the obtained platinum complex [1c''] is allowed to react with a halogenating agent for substituting a desirable halogen atom in the presence of an appropriate solvent, if required, under an inert gas atmosphere.

In this case, by directly adding the halogenating agent in the reaction system of the platinum diene complex [2b] and the compound [3] without performing a post treatment such as isolation, and the like on the platinum complex [1c"] obtained by allowing the platinum diene complex [2b] and the compound [3] to react with other, i.e., by effecting the reaction in one pot, it is possible to produce the platinum complex [1d''] more efficiently and easily. Incidentally, the halogenating agent may be added into the reaction system as it is, or dissolved in a solvent, and added therein. Further, a solvent may be added, if required.

The kind of the halogenating agent and the preferred halogenating agent for use in this production method are as described previously.

The amount of the halogenating agent to be used is appropriately selected from the range of generally 1 to 100 equivalents, preferably 1 to 20 equivalents, and more preferably 1 to 10 equivalents relative to the platinum diene complex [1c''].

Incidentally, the halogenating agent herein used is a halogenating agent for substituting a desirable halogen atom as described above. Therefore, as a matter of course, X ≠ X³. Namely, when X of the platinum diene complex [2b] is Cl (chlorine atom), a halogenating agent (such as fluoride, bromide, or iodide) other than a chlorinating agent (chloride) is used as the halogenating agent. Similarly, when X = F (fluorine atom), a halogenating agent (such as chloride, bromide, or iodide) other than a fluorinating agent (fluoride) is used as the halogenating agent; when X = Br (bromine atom), a halogenating agent (such as fluoride, chloride, or iodide) other than a brominating agent (bromide), as the halogenating agent; and when X = I (iodine atom), a halogenating agent (such as fluoride, chloride, or bromide) other than an iodinating agent (iodide), as the halogenating agent.

The amounts of the platinum diene complex [2b] and the compound [3] are the same as those for the production method 1". Further, the type of the solvent is also the same as that for the production method 1''.

The amount of the solvent to be used has no particular restriction so long as it is such an amount as to allow the reaction to proceed sufficiently. The amounts of the solvent to be used when three of the platinum diene complex [2b], the compound [3], and the halogenating agent are mixed and allowed to mutually react, and when the platinum diene complex [2b] and the compound [3] are first allowed to react with each other are the same as those for the production method 1". Whereas, when the platinum complex [1c''] has been once isolated, the amount of the solvent in the subsequent reaction is appropriately selected from the range of generally 1- to 200-fold volume, and preferably 1- to 50-fold volume relative the platinum complex [1c''].

The reaction temperature and the reaction time when three of the platinum diene complex [2b], the compound [3], and the halogenating agent are mixed and allowed to mutually react, and when the platinum diene complex [2b] and the compound [3] are first allowed to react with each other may be the same as those for the production method 1''.

Whereas, the reaction temperature for the platinum complex [1c''] and the halogenating agent when the platinum complex [1c''] has been once isolated is appropriately selected from the range of generally 25 to 300 °C, preferably 60 to 200 °C, and more preferably 80 to 150 °C. The reaction time is appropriately selected from the range of generally 10 minutes to 72 hours, preferably 30 minutes to 48 hours, and more preferably 1 to 12 hours.

Further, the reaction time when the reaction of the platinum diene complex [2b] and the compound represented as the compound [3], and the subsequent reaction with the halogenating agent are effected in one pot is desirably as follows. After 10 minutes to 72 hours, preferably 30 minutes to 48 hours, and more preferably 1 hour to 12 hours from the start of the reaction of the platinum diene complex [2b] and the compound [3], the halogenating agent is added into the reaction system. Then, the reaction is effected generally for 10 minutes to 72 hours, preferably 30 minutes to 48 hours, and more preferably 1 to 12 hours.

Any process of the production method 2" may be accomplished by effecting the reaction, if required, using an ultrasonic generator in combination.

Whereas, any process of the production method 2" is preferably carried out under an inert gas atmosphere. As the inert gas, mention may be made of the same ones for the production method 1''.

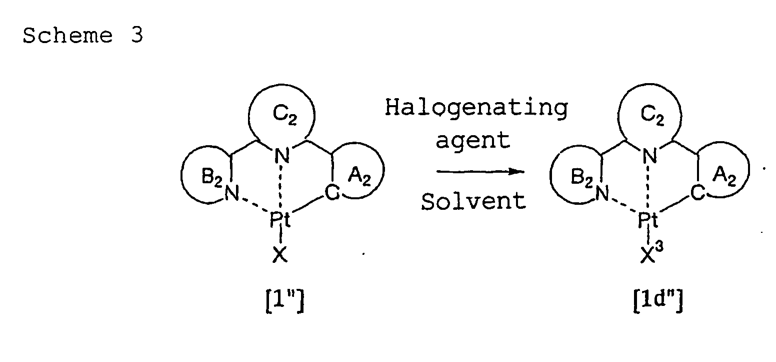

The scheme 3 is a formula for explaining the production method of the platinum complex [1d''] (the platinum complex [1c''] having a desirable halogen atom) using the platinum complex [1c''] as a raw material compound (which is hereinafter abbreviated as the "production method 3''").

The platinum complex [1d''] can be produced with ease in the following manner. The platinum complex [1c"] obtained with the production method 1" is used. This is allowed to react with a halogenating agent for substituting a desirable halogen atom in the presence of an appropriate solvent, if required, under an inert gas atmosphere.

The type of the halogenating agent and the amount thereof, the halogenating agent to be used, the kind of the solvent and the amount thereof, the reaction temperature, and the reaction time are the same as those for the halogenating step in the production method 2''.

The production method 3'' may also be accomplished by effecting the reaction, if required, using an ultrasonic generator in combination.

Whereas, the production method 3" is also preferably carried out under an inert gas atmosphere. As the inert gas, mention may be made of the same ones for the production method 1".

In the production method 3", for example, when the platinum complex of the general formula [1c''] where X is chlorine and a halogenating agent for substituting a halogen other than chlorine are allowed to react with each other, it is possible to produce a platinum complex represented by the general formula [1'']: 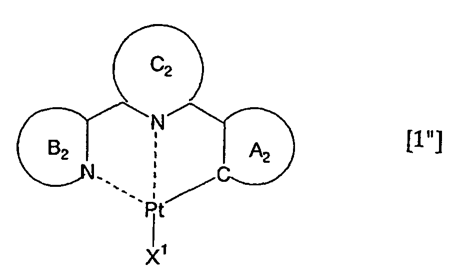 (where in the formula, the ring B₂, the ring C₂, and the ring A₂ are the same as described above, and X¹ represents a fluorine atom, a bromine atom, or an iodine atom).

Incidentally, the platinum complexes represented by the general formula [1''] are all novel compounds.

As the platinum diene complex [2b] and the compound [3] for use in the production methods 1" to 3", commercially available products may be used, or those appropriately produced may be used.

The platinum complex thus obtained may be subjected to, if required, post treatments, isolation and purification. Examples of the post treatment may include extraction of the reactant, filtration of the precipitate, crystallization by addition of a solvent, and distilling away of the solvent. These post treatments may be carried out alone, or appropriately in combination. Examples of the method of isolation and purification may include column chromatography, recrystallization, and sublimation. These may be carried out alone, or appropriately in combination.

On the other hand, the platinum complex of the invention represented by the general formula [1'''] can be obtained in the following manner. For example, the platinum diene complex represented by the general formula [2b] and the compound represented by the general formula [3] are allowed to react with each other, resulting in the platinum complex represented by the general formula [1c'']. Then, on this, a Grignard reagent represented by the general formula [4]:

EMgX² [4]

(where in the formula, E represents an aryl group which may be substituted or a heteroaryl group which may be substituted, and X² represents a halogen atom) is allowed to act.

The platinum complex of the invention represented by the general formula [1"'] can also be obtained in the following manner. For example, a platinum compound represented by the general formula [5]:

M₂PtX₄ [5]

(where in the formula, M represents an alkali metal atom, and X represents a halogen atom) and the compound represented by the general formula [3] are allowed to react with each other, resulting in the platinum complex represented by the general formula [1c'']. Then, on this, the Grignard reagent represented by the general formula [4] is allowed to act.

Still further, the platinum complex of the invention represented by the general formula [1'''] can also be obtained in the following manner. For example, on the platinum diene complex represented by the general formula [2b], the Grignard reagent represented by the general formula [4] is allowed to act. Then, the compound represented by the general formula [3] is allowed to react with this.

The platinum complex of the invention represented by the general formula [1'''] can be produced, for example, by the following production methods 1''' to 3''', and the like.

### Production method 1'''

A platinum diene complex represented by the general formula [2b] such as dichloro(1,5-hexadiene) platinum and a compound represented by the general formula [3], such as 6-phenyl-2,2'-bipyridine are allowed to react with each other with stirring in a solvent such as 2-ethoxyethanol or acetonitrile, at a reaction temperature of 50 °C to 150 °C, and preferably 80 °C to 120 °C, for 1 hour to several days, and preferably for 2 hours to one day. As a result, a platinum complex represented by the general formula [1c"] such as chloro(6-phenyl-2,2'-bipyridine)platinum is obtained. Then, a Grignard reagent represented by the general formula [4] such as phenylmagnesium bromide is allowed to react with this at a reaction temperature of 0 °C to 100 °C, and preferably 20 °C to 80 °C, for 30 minutes to 4 hours, and preferably for 1 to three hours. As a result, a platinum complex of the invention represented by the general formula [1'''] such as [6-phenyl-2,2'-bipyridinato(C, N, N)]phenyl platinum (II) can be obtained. These reactions are preferably effected in an inert gas such as nitrogen or argon.

### Production method 2'''

A platinum compound represented by the general formula [5] such as potassium tetrachloroplatinate and a compound represented by the general formula [3], such as 6-phenyl-2,2'-bipyridine are allowed to react with each other in a solvent such as acetic acid, at a reaction temperature of 50 °C to 150 °C, and preferably 80 °C to 120 °C, for 1 hour to several days, and preferably for 2 hours to two days. As a result, a platinum complex represented by the general formula [1c''] such as chloro(6-phenyl-2,2'-bipyridine)platinum is obtained. Then, a Grignard reagent represented by the general formula [4] is allowed to react with this in the same manner as with the production method 1'''. As a result, a platinum complex of the invention represented by the general formula [1'''] such as [6-phenyl-2,2'-bipyridinato(C, N, N)]phenyl platinum (II) can be obtained. These reactions are also preferably effected in an inert gas such as nitrogen or argon.

### Production method 3'''

A platinum diene complex represented by the general formula [2b] such as dichloro(1,5-cyclooctadiene) platinum is allowed to react with a Grignard reagent represented by the general formula [4] such as phenylmagnesium bromide at a reaction temperature of 0 °C to 100 °C, and preferably 20 °C to 80 °C for 30 minutes to 4 hours, and preferably for 1 to 3 hours. As a result, chloro(aryl) (1,5-cyclooctadiene)platinum is obtained. Then, the resulting compound is allowed to react with a compound represented by the general formula [3] such as 6-phenyl-2,2'-bipyridine with stirring at a reaction temperature of 30 °C to 200 °C, and preferably 80 °C to 110 °C, for 2 hours to 5 days, and preferably for 18 hours to 3 days. As a result, a platinum complex of the invention represented by the general formula [1'''] can be obtained. These reactions are also preferably effected in an inert gas such as nitrogen or argon.

Incidentally, a platinum diene complex represented by the general formula [2b] can be obtained with ease in the following manner. For example, a platinum compound such as potassium tetrachloroplatinate and dienes such as 1, 5-hexadiene and 1, 5-cyclooctadiene are heated with stirring in a solvent such as acetic acid, 2-ethoxyethanol, or acetonitrile at a reaction temperature of 50 °C to 140 °C for 15 minutes to 3 hours.

As the raw material platinum compound herein used, mention may be made of, other than potassium tetrachloroplatinate, potassium tetrabromoplatinate, sodium tetrachloroplatinate, or the like. Alternatively, a hydrate thereof may be used. Whereas, as the reaction solvent, acetic acid, 2-ethoxyethanol, acetonitrile, or the like can be used. The solvent may be mixed with water to serve as a hydrous solvent for effecting the reaction.

The platinum complexes of the invention, and the platinum complexes obtained with the production methods of the invention can be effectively used as, for example, light emitting materials of light emitting devices, particularly, as phosphorescent materials, or electric charge transport materials, and the like. Incidentally, as typical light emitting devices, mention may be made of organic EL devices.

Below, a description will be given to examples in which the platinum complexes of the invention, and the platinum complexes obtained with the production method of the invention (which are hereinafter simply referred to as the platinum complexes in accordance with the invention) are used as light emitting devices.

So long as the light emitting devices in accordance with the invention are the light emitting devices utilizing the platinum complexes in accordance with the invention, the system, the driving method, the use form, and the like do not particularly matter. However, the ones utilizing luminescence from the platinum complexes in accordance with the invention, and the ones utilizing the platinum complexes in accordance with the invention as electric charge transport materials are preferred. As typical light emitting devices, mention may be made of organic EL devices.

It is only essential that the light emitting devices containing the platinum complexes in accordance with the invention contain at least one of the platinum complexes. In a light emitting device in which a light emitting layer or a plurality of organic compound layers including a light emitting layer are formed between a pair of electrodes, at least one layer contains at least one of the platinum complexes. The platinum complexes may be contained therein at least singly, or may be contained in appropriate combination of two or more thereof.

The method for forming the organic layer (organic compound layer) of the light emitting device containing the platinum complexes in accordance with the invention has no particular restriction. However, methods of resistance heating deposition, electron beam, sputtering, a molecular lamination method, a coating method, an ink jet method, and the like are used. The resistance heating deposition and coating methods are preferred from the viewpoints of the characteristics and production.

The light emitting device containing the platinum complex in accordance with the invention is a device in which a light emitting layer or a plurality of organic compound thin layers including a light emitting layer are formed between a pair of an anode and a cathode. It may have, other than the light emitting layer, a hole injection layer, a hole transport layer, an electron injection layer, an electron transport layer, a protective layer, and the like. Further, these respective layers may be the ones respectively having other functions. Various materials may be respectively used for the formation of the respective layers.

The anode is the one for supplying holes to the hole injection layer, the hole transport layer, the light emitting layer, and the like. A metal, an alloy, a metal oxide, an electrically conductive compound, or a mixture thereof can be used. A material with a work function of 4 eV or more is preferred. Specific examples thereof may include conductive metal oxides such as tin oxide, zinc oxide, indium oxide, indium tin oxide (which is hereinafter abbreviated as ITO), or metals such as gold, silver, chromium, and nickel, further, mixtures or laminated products of these metals and conductive metal oxides, inorganic conductive substances such as copper iodide and copper sulfide, organic conductive materials such as polyaniline, polythiophene, and polypyrrole, and laminated products of these and ITO. The conductive metal oxides are preferred, and ITO is particularly preferred from the viewpoints of productivity, high conductivity, transparency, and the like. The film thickness of the anode can be appropriately selected according to the material. In general, it is preferably in the range of 10 nm to 5 µm, more preferably 50 nm to 1 µm, and further preferably 100 nm to 500 nm.

As the anode, the one formed in a layer on a soda lime glass, non-alkali glass, or transparent resin substrate, or the like is generally used. When glass is used, as the material, non-alkali glass is preferably used in order to reduce the ions dissolved from glass. Whereas, when soda lime glass is used, the one barrier-coated with silica or the like is preferably used. The thickness of the substrate has no particular restriction so long as it is enough for keeping the mechanical strength. When glass is used, a substrate with a thickness of generally 0.2 mm or more, and preferably 0.7 mm or more is used. For the production of the anode, various methods are used according to the material. For example, in the case of ITO, the film formation is carried out with a method such as an electron beam method, a sputtering method, a resistance heating deposition method, a chemical reaction method (such as a sol-gel method), or coating of an ITO dispersion. For the anode, a washing or other treatment can also reduce the driving voltage of the device, and increase the luminous efficiency. For example, in the case of ITO, a UV-ozone treatment, a plasma treatment, or the like is effective.

The cathode is the one for supplying electrons to the electron injection layer, the electron transport layer, the light emitting layer, and the like. It is selected in consideration of the adhesion with the layer adjacent to the negative electrode such as the electron injection layer, the electron transport layer, or the light emitting layer, the ionization potential, the stability, and the like. As the material for the cathode, a metal, an alloy, a metal halide, a metal oxide, an electrically conductive compound, or a mixture thereof can be used. Specific examples thereof may include alkali metals such as lithium, sodium, and potassium, and fluorides thereof, alkaline earth metals such as magnesium and calcium, and fluorides thereof, gold, silver, lead, aluminum, and sodium-potassium alloys, or mixed metals thereof, magnesium-silver alloys or mixed metals thereof, and rare earth metals such as indium and ytterbium. The materials with a work function of 4 eV or less are preferred, and aluminum, lithium-aluminum alloys or mixed metals thereof, magnesium-silver alloys or mixed metals thereof, and the like are more preferred.

The cathode can also assume a laminated structure containing the compound and mixture. The film thickness of the cathode can be appropriately selected according to the material. In general, it is preferably in the range of 10 nm to 5 µm, more preferably 50 nm to 1 µm, and further preferably 100 nm to 1 µm. For fabrication of the cathode, a method such as an electron beam method, a sputtering method, a resistance heating deposition method, or a coating method is used. Metals can be vapor deposited in the form of a simple substance, or two or more components thereof may be simultaneously vapor deposited. Further, it is also possible to simultaneously vapor deposit a plurality of metals for forming the pole with an alloy. Alternatively, a previously prepared alloy may be vapor deposited. The sheet resistance of the cathode and the anode is preferably lower.

Any material for the light emitting layer is acceptable so long as it can form the layer having a function of capable of injecting electrons from the anode or the hole injection layer, or the hole transport layer under an applied electric field, and a function of providing a site for recombination of holes and electrons, and effecting luminescence. It is possible to dope a fluorescent material and a phosphorescent material having a high luminous efficiency in the light emitting layer. Examples thereof may include various metal complexes typified by metal complexes and rare earth complexes of a benzoxazole derivative, a triphenylamine derivative, a benzimidazole derivative, a benzothiazole derivative, a styrylbenzene derivative, a polyphenyl derivative, a diphenylbutadine derivative, a tetraphenylbutadiene derivative, a naphthalimide derivative, a coumarin derivative, a perylene derivative, a perynone derivative, an oxadiazole derivative, an aldazine derivative, a pyralidine derivative, a cyclopentadiene derivative, a bisstyrylanthracene derivative, a quinacridone derivative, a pyrrolopyridine derivative, thiadiazopyridine derivative, a styrylamine derivative, an aromatic dimethylidene compound, and a 8-quinolinol derivative, polymer compounds such as polythiophene, polyphenylene, and polyphenylenevinylene, an organic silane derivative, and the platinum complexes in accordance the invention. The layer may be formed in a monolayered structure composed of one, or two or more of the foregoing materials, or may be formed in a multilayered structure composed of a plurality of layers of the same composition or different kind of compositions. The film thickness of the light emitting layer has no particular restriction. However, in general, it is preferably in the range of 1 nm to 5 µm, more preferably 5 nm to 1 µm, and further preferably 10 nm to 500 nm. The method for fabricating the light emitting layer has no particular restriction. However, a method such as an electron beam method, a sputtering method, a resistance heating deposition method, a molecular lamination method, a coating method (such as a spin coating method, a casting method, or a dip coating method), an ink jet method, or an LB method is used. The resistance heating deposition and coating methods are preferred.

Any material for the hole injection layer and the hole transport layer is acceptable so long as it has any of a function of injecting holes from the anode, a function of transporting holes, and a function of barriering the electrons injected from the cathode. Specific examples thereof may include a carbazole derivative, a triazole derivative, an oxadiazole derivative, an oxazole derivative, an imidazole derivative, a polyarylalkane derivative, a pyrazoline derivative, a pyrazolone derivative, a phenylenediamine derivative, an arylamine derivative, an amino-substituted chalcone derivative, a styrylanthracene derivative, a fluorenone derivative, a hydrazone derivative, a stilbene derivative, a silazane derivative, an aromatic tertiary amine compound, a styrylamine compound, an aromatic dimethylidine type compound, a porphyrin type compound, a polysilane type compound, a poly(N-vinyl carbazole) derivative, an aniline type copolymer, a thiophene oligomer, a conductive polymer oligomer such as polythiophene, an organic silane derivative, and the platinum complexes in accordance with the invention. The film thickness of the hole injection layer or the hole transport layer has no particular restriction. However, in general, it is preferably in the range of 1 nm to 5 µm, more preferably 5 nm to 1 µm, and further preferably 10 nm to 500 nm. The hole injection layer or the hole transport layer may be formed in a monolayered structure composed of one, or two or more of the foregoing materials, or may be formed in a multilayered structure composed of a plurality of layers of the same composition or different kind of compositions. As the method for fabricating the hole injection layer or the hole transport layer, a method such as a vacuum deposition method, an LB method, a method in which the hole injection transport agent is dissolved or dispersed in a solvent for coating (such as a spin coating method, a casting method, or a dip coating method), or an ink jet method is used. For the coating method, dissolution or dispersion with a resin component is possible. Examples of the resin component may include polyvinyl chloride, polycarbonate, polystylene, polymethyl methacrylate, polybutyl methacrylate, polyester, polysulfone, polyphenylene oxide, polybutadiene, poly(N-vinyl carbazole), a hydrocarbon resin, a ketone resin, a phenoxy resin, polyamide, ethyl cellulose, vinyl acetate, an ABS resin, an alkyd resin, an epoxy resin, and a silicon resin.

Any material for the electron injection layer and the electron transport layer is acceptable so long as it has any of a function of injecting electrons from the cathode, a function of transporting electrons, and a function of barriering the holes injected from the anode. The ionization potential of the hole blocking layer having a function of barriering the holes injected from the anode is selected to be larger than the ionization potential of the light emitting layer.

Specific examples thereof may include various metal complexes typified by metal complexes of a triazole derivative, an oxazole derivative, a polycyclic type compound, a heteropolycyclic compound such as bathocuproin, an oxadiazole derivative, a fluorenone derivative, a diphenylquinone derivative, a thiopyran dioxide derivative, an anthraquinonedimethane derivative, an anthrone derivative, a carbodiimide derivative, a fluorenylidenemethane derivative, a distyrylpyrazine derivative, tetracarboxylic acid anhydrides of aromatic rings such as naphthalene and perylene, a phthalocyanine derivative, and a 8-quinolinol derivative, metal phthalocyanine, and a metal complex having benzoxazole or benzothiazole as a ligand, an organosilane derivative, and the platinum complexes in accordance with the invention. The film thickness of the electron injection layer or the electron transport layer has no particular restriction. However, in general, it is preferably in the range of 1 nm to 5 µm, more preferably 5 nm to 1 µm, and further preferably 10 nm to 500 nm. The electron injection layer or the electron transport layer may be formed in a monolayered structure composed of one, or two or more of the foregoing materials, or may be formed in a multilayered structure composed of a plurality of layers of the same composition or different kind of compositions. As a method for forming the electron injection layer or the electron transport layer, a method such as a vacuum deposition method, an LB method, a method in which the hole injection transport agent is dissolved or dispersed in a solvent for coating (such as a spin coating method, a casting method, or a dip coating method), or an ink jet method is used. For the coating method, dissolution or dispersion with a resin component is possible. As the resin components, the ones listed in the case of the hole injection layer and the hole transport layer are applicable.

Any material for the protective layer is acceptable so long as it has a function of inhibiting the one promoting the device deterioration such as moisture or oxygen from entering the device. Specific examples thereof may include metals such as indium, tin, lead, gold, silver, copper, aluminum, titanium, and nickel, metal oxides such as magnesium oxide, silicon oxide, dialuminum trioxide, germanium oxide, nickel oxide, calcium oxide, barium oxide, diiron trioxide, diytterbium trioxide, and titanium oxide, metal fluorides such as magnesium fluoride, lithium fluoride, aluminum fluoride, and calcium fluoride, polyethylene, polypropylene, polymethyl methacrylate, polyimide, polyurea, polytetrafluoroethylene, polychlorotrifluoroethylene, polydichlorodifluoroethylene, a copolymer of chlorotrifluoroethylene and dichlorodifluoroethylene, a copolymer obtained by polymerizing a monomer mixture containing tetrafluoroethylene and at least one comonomer, a fluorine-containing copolymer having a cyclic structure on the copolymer main chain, a water-absorbing substance having a water absorption of 1 % or more, and a moisture-proof substance having a water absorption of 0.1 % or less. The method for forming the protective layer has no particular restriction. For example, a vacuum deposition method, a sputtering method, a reactive sputtering method, a MBE (molecular beam epitaxy) method, a cluster ion beam method, an ion plating method, a plasma polymerization method (high frequency excitation ion plating method), a plasma CVD method, a laser CVD method, a heat CVD method, a gas source CVD method, or a coating method is applicable.

### Examples

Below, the present invention will be described in details by way of reference examples, examples, comparative examples, and use examples. However, the invention is not limited by these at all.

Incidentally, in the following examples and the like, the apparatuses used for the measurements of the physical properties are as follows:
1) ¹H-NMR Spectrum: GEMINI 2000 model apparatus (manufactured by VARIAN Inc.) or DRX-500 model apparatus (manufactured by BRUKER Co.) Internal standard substance: tetramethylsilane
2) Organic elementary analysis: CHN coder MT-5 model apparatus (manufactured by YANAGIMOTO Co., Ltd.)
3) Absorption spectrum analysis: V-550 (manufactured by JASCO)
4) Luminescence emission spectrum analysis: F-4500 (manufactured by HITACHI)

Whereas, the purity of the product is 100 % unless otherwise specified in the description.

### Reference Example 1 Production of 1,3-di(2-pyridyl)benzene

The production was accomplished according to the following reaction formula in accordance with the description of Organometallics 1999, 18, p3337 to p3341).

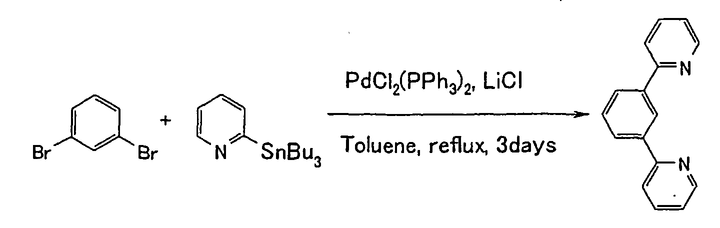

2.5 g of 1,3-dibromobenzene, 7.9 g of (2-pyridyl) tributylstannane, 0.60 g of bis (triphenylphosphine) dichloropalladium, 0.45 g of lithium chloride and 25 ml of toluene were mixed. The resulting mixture was stirred under reflux for 3 days. It was cooled to room temperature, followed by washing with water, and purified by a silica gel column chromatography. As a result, 1.4 g of the subject target compound was obtained as a colorless liquid.
¹H-NMR(CDCl₃) :δ 7.22-7.29(m,2H),7.59 (t, J=7.8Hz, 1H), 7. 73-7.87(m,4H),8.07 (dd, J=1.8Hz, 7.8Hz, 2H), 8.61-8.76(m, 3H).

### Reference Example 2 Production of 1-fluoro-3,5-di(2-pyridyl)benzene

In the same manner as with Reference Example 1, 1.4 g of 1-fluoro-3,5-dibromobenzene, 5.0 g of (2-pyridyl)tributylstannane, 0.38 g of bis(triphenylphosphine)dichloropalladium, 0.28 g of lithium chloride and 14 ml of toluene were mixed. The resulting mixture was stirred under reflux for 3 days. It was cooled to room temperature, followed by washing with water, and purified by a silica gel column chromatography. As a result, 1.2 g of the subject target compound was obtained as a white solid.
¹H-NMR(CDCl₃) :δ 7.22-7.32(m, 2H), 7.77-7.83(m, 4H), 8.44(t, J=1.4Hz, 1H), 8.73(dt, J=4.8Hz,1.6Hz,2H).

### Reference Example 3 Production of 1-methoxy-2,4-di(2-pyridyl)benzene

In the same manner as with Reference Example 1, 5.0 g of 2,4-dibromoanisole, 6.9 g of (2-pyridyl)tributylstannane, 1.0 g of bis(triphenylphosphine)dichloropalladium, 0.8 g of lithium chloride and 50 ml of toluene were mixed. The resulting mixture was stirred under reflux for 3 days. It was cooled to room temperature, followed by washing with water, and purified by a silica gel column chromatography. As a result, 2.9 g of the subject target compound was obtained as a colorless liquid.
¹H-NMR(CDCl₃) :δ 3.92(s,3H),7.09-7.27 (m,3H),7.67-7.87 (m, 4H), 8.11(dd, J=2.6Hz, 8.6Hz, 1H), 8.37(d, J=2.4Hz, 1H), 8.64 -8.74(m,2H).

### Example 1 Production of platinum complex (1'-1)

0. 28 g of 1, 3-di (2-pyridyl) benzene produced in Reference Example 1, and 0.5 g of potassium tetrachloroplatinate were mixed in 10 ml of acetic acid. The resulting mixture was stirred at 80 °C for 3 days. It was cooled to room temperature, and then, the precipitated solid was filtered off, and dried, resulting in 0.44 g of a dihydrate of the platinum complex (1'-1) in the form of a yellow solid.

Elementary analysis: calculated values (C, 38.60; H, 3.04; N, 5.63).measured values (C, 38, 27; H, 2.94; N, 5.50).

0.20 g of the dihydrate the resulting compound (1'-1), and 1.3 ml of a tetrahydrofuran solution (2.00 M) of ethylmagnesium chloride were mixed in 10 ml of tetrahydrofuran. The resulting mixture was stirred at room temperature for 2 hours. The reaction solution was extracted with methylene chloride. Then, a neutral silica gel column chromatography (methylene chloride / methanol = 1/0 to 20/1) was carried out, resulting in 0.10 g of a target compound (1'-1) in the form of a yellow solid.

Elementary analysis: calculated values (C, 41.61; H, 2.40; N, 6.07). measured values (C, 41.58; H, 2.35; N, 5.94).

### Example 2 Production of platinum complex (1'-2)

0.87 g of the dihydrate of the platinum complex (1'-1) obtained in the first half of Example 1, and 9 ml of a tetrahydrofuran solution (1.04 M) of phenylmagnesium bromide were mixed in 22 ml of tetrahydrofuran. The resulting mixture was heated under reflux for 3 hours. After cooling to room temperature, the reaction solution was extracted with methylene chloride. Then, a neutral silica gel column chromatography (methylene chloride / methanol = 1/0 to 20/1) was carried out, resulting in 0.26 g of a target platinum complex (1'-2) in the form of a yellow solid.
¹H-NMR(DMSO-d₆) :δ 7.32 (t, J=7.7Hz, 1H), 7.52-7.55(m, 2H), 7.76(d, J=7.7Hz, 2H), 8.10-8.12(m, 2H), 8.19(dt, J=1.6Hz, 7.9Hz, 2H), 9.27-9.40 (m, 2H).

Elementary analysis: calculated values (C, 37.96; H, 2. 19; N, 5.53%). measured values (C, 37.89; H, 2.13; N, 5.13%).

The solution fluorescence spectrum of this compound was measured. As a result, it was found that λmax of fluorescence = 491.4 nm, 523.6 nm (CH₂Cl₂).

### Example 3 Production of platinum complex (1'-3)

20 ml of a tetrahydrofuran solution of 1.1 g of iodobenzene was added dropwise to 0.13 g of magnesium to produce phenylmagnesium iodide. To the reaction solution, 0.44 g of the dihydrate of the platinum complex (1'-1) obtained in the first half of Example 1 was added. The resulting mixture was stirred under reflux for 3 hours. After cooling to room temperature, the reaction solution was extracted with methylene chloride. Then, a neutral silica gel column chromatography (methylene chloride / methanol = 1/0 to 20/1) was carried out, resulting in 0.10 g of a target compound (1'-3) in the form of a yellow solid.

Elementary analysis: calculated values (C,34.73; H, 2.00; N, 5.06%). measured values (C, 34.70; H, 2.25; N, 4.96 %).

### Example 4 Production of platinum complex (1'-5)

0. 41 g of 1-fluoro-3, 5-di (2-pyridyl) benzene produced in Reference Example 2, and 0.68 g of potassium tetrachloroplatinate were mixed in 14 ml of acetic acid. The resulting mixture was stirred at 90 °C for 3 days. It was cooled to room temperature, and then, the precipitated solid was filtered off, and dried, resulting in 0.56 g of a yellow solid.

0.40 g of the resulting yellow solid, and 2.4 ml of a tetrahydrofuran solution (1.04 M) of phenylmagnesium bromide were mixed in 8 ml of tetrahydrofuran. The resulting mixture was stirred for 3 hours. The reaction solution was extracted with methylene chloride. Then, a neutral silica gel column chromatography (methylene chloride / methanol = 1/0 to 20/1) was carried out, resulting in 0.11 g of a target platinum complex (1'-5) in the form of a yellow solid.
¹H-NMR(DMSO-d₆) :δ 7.56-7.59(m,2H),7.77(d, J=10.3Hz,2H), 8.15-8.24(m,4H),9.27-9.43(m,2H).

### Example 5 Production of platinum complex (1b''-1) ((6-phenyl-2,2'-bipyridinato-C,N,N)platinum (II) chloride)

[(1,2,5,6-η⁴)-1,5-hexadienyl]platinum(II) dichloride (500 mg, 1.44 mmol, 1.0 equivalent) and 6-phenyl-2,2'-bipyridyl (400 mg, 1.72 mmol, 1.2 equivalents) were charged into a schlenk flask, whose inside was replaced with nitrogen. Then, 2-ethoxyethanol (10 mL) was added thereto, and the mixture was heated, and allowed to react with stirring under reflux for 1 hour. Then, the solvent was distilled off under reduced pressure. The resulting red orange residue was purified with a silica gel column chromatography (eluent: dichloromethane / methanol = 20/1), and then recrystallized from hexane / dichloromethane. As a result, 648 mg of the platinum complex (1b''-1) was obtained as an orange powder. Yield 97.4 %.
¹H-NMR (500MHz, CD₂Cl₂) :δ 7.10 (dt, J=1.4, 7.5Hz, 1H), 7.16-7. 24(m, 1H), 7.38(dd, J=1.4, 7.5Hz, 1H), 7.50-7.65(m, 3H), 7.68(ddd, J=1.3Hz, 5.3, 7.9Hz, 1H), 7.88(t, J=8.0Hz, 1H), 7.94(dt, J=7.9, 0.9 Hz, 1H), 8.09(dt, J=1.6, 7.9Hz, 1H), 9.04-9.09(m, 1H).
Excitation wavelength: 330.0 nm, Fluorescence wavelength: 559.4 nm.

### Example 6 Production of platinum complex (1b''-5) ((6,6'-diphenyl-2,2'-bipyridinato-C,N,N)platinum (II) chloride)

[(1,2,5,6-η⁴)-1,5-hexadienyl]platinum(II) dichloride (500 mg, 1.44 mmol, 1.0 equivalent) and 6,6'-diphenyl-2,2'-bipyridyl (534 mg, 1.72 mmol, 1.2 equivalents) were charged into a schlenk flask, whose inside was replaced with nitrogen. Then, 2-ethoxyethanol (25 mL) was added thereto, and the mixture was heated, and allowed to react with stirring under reflux for 4 hours. Then, the solvent was distilled off under reduced pressure. The resulting red orange residue was purified with a silica gel column chromatography (eluent: dichloromethane), and then recrystallized from hexane / dichloromethane. As a result, 597 mg of the platinum complex (1b''-5) was obtained as an orange powder. Yield 77.1 %.
¹H-NMR(500MHz, CD₂Cl₂) :δ 7.07(dt, J=1.3, 7.5Hz, 1H), 7.14 (dt, J=1.5, 7.5Hz, 1H), 7.38(dd, J=1.5, 7.5Hz, 1H), 7.46-7.55(m, 3H), 7.56-7.64(m, 2H), 7.68-7.74(m, 3H), 7.75(dd, J=1.3, 7.9Hz, 1 H), 7.95(t, J=8.1Hz, 1H), 8.00(dd, J=1.3, 7.9Hz, 1H), 8.09(t, J= 7.9Hz, 1H).

### Example 7 Production of platinum complex (1b''-9) ((2,9-diphenyl-1,10-phenanthrolinato-C,N,N)platinum (II) chloride)

[(1,2,5,6-η⁴)-1,5-hexadienyl]platinum(II) dichloride (500 mg, 1.44 mmol, 1.0 equivalent) and 2,9-diphenyl-1,10-phenanthroline (574 mg, 1.72 mmol, 1.2 equivalents) were charged into a schlenk flask, whose inside was replaced with nitrogen. Then, 2-ethoxyethanol (10 mL) was added thereto, and the mixture was heated, and allowed to react with stirring under reflux for 3 hours. Then, the solvent was distilled off under reduced pressure. The resulting red orange residue was purified with a silica gel column chromatography (eluent: dichloromethane / methanol = 100/1), and then recrystallized from hexane / dichloromethane. As a result, 706 mg of the platinum complex (1b''-9) was obtained as a red orange powder. Yield 87.2 %.
¹H-NMR(500MHz CD₂Cl₂) :δ 7.11 (dt, J=1.3, 7.4Hz, 1H), 7.16-7.24(m, 1H), 7.49-7.58(m, 4H), 7.63-7.77(m, 1H), 7.80-7.91(m, 5H), 7.96(d, J=8.5Hz, 1H), 8.42(d, J=8.6Hz, 1H), 8.50(d, J=8.5Hz, 1 H).

### Example 8 Production of platinum complex (1b''-15) ((2,4,7,9-tetraphenyl-1,10-phenanthrolinato-C,N,N)platinum (II) chloride)

[(1,2,5,6-η⁴)-1,5-hexadienyl]platinum(II) dichloride (500 mg, 1.44 mmol, 1.0 equivalent) and 2,4,7,9-tetraphenyl-1,10-phenanthroline (837 mg, 1.72 mmol, 1.2 equivalents) were charged into a schlenk flask, whose inside was replaced with nitrogen. Then, 2-ethoxyethanol (25 mL) was added thereto, and the mixture was heated, and allowed to react with stirring under reflux for 3 hours. Then, the solvent was distilled off under reduced pressure. The resulting red orange residue was purified with a silica gel column chromatography (eluent: dichloromethane / methanol = 100/1), and then recrystallized from hexane / dichloromethane. As a result, 988 mg of the platinum complex (1b"-15) was obtained as a red orange powder. Yield 96.1 %.
¹H-NMR(500MHz, CD₂Cl₂) :δ 7.10 (dt, J=1.3, 7.5Hz, 1H), 7.20 (dt, J=1.5, 7.5Hz, 1H), 7.50-7.80(m, 15H), 7.83(s, 1H), 7.85-7.92 (m, 4H), 7.95(s, 1H).

### Comparative Example 1 Production of platinum complex (1b"-15)

### ((2,4,7,9-tetraphenyl-1,10-phenanthrolinato-C,N,N)platinum (II) chloride) by a known method (J. Chem. Soc. Dalton Trans. , 1996, 1645-1651)

Potassium tetrachloroplatinate (II) (78 mg, 0.187 mmol, 1.0 equivalent) and 2,4,7,9-tetraphenyl-1,10-phenanthroline (100 mg, 0.206 mmol, 1.1 equivalents) were charged into a schlenk flask, whose inside was replaced with nitrogen. Then, 2-ethoxyethanol (10 mL) and water (5 mL) were added thereto, and the mixture was heated. At the instant when the internal temperature had reached 80 °C, a black precipitate which can be considered to be derived from platinum (0) was gradually formed. The reaction solution was further stirred for 3 hours under reflux for 3 hours. Then, the solvent was distilled off under reduced pressure. The resulting black residue was purified with a silica gel column chromatography (eluent: dichloromethane / methanol = 100/1), and then the column fraction condensate was washed with hexane. As a result, 13 mg of the platinum complex (1b''-15) was obtained as a red orange powder. Purity 97.9 %, Yield 9.5 %. The physical property values of ¹H-NMR and the like were in accordance with those for the platinum complex (1b"-15) obtained in Example 8.

### Comparative Example 2 Production of platinum complex (1b"-15)

### ((2,4,7,9-tetraphenyl-1,10-phenanthrolinato-C,N,N)platinum (II) chloride) by a known method (J. Chem. Soc. Dalton Trans., 1990, 443-449, and the like)

The reaction was effected in the same manner as with Comparative Example 1, except that acetonitrile was used in place of 2-ethoxyethanol in Comparative Example 1. However, it was not possible to obtain a target platinum complex (1b"-15).

As apparent also from the results of Example 8, and Comparative Examples 1 and 2, it is indicated that the production method of the invention is apparently excellent.

### Example 9 Production of platinum complex (1"-2) ((6-phenyl-2,2'-bipyridinato-C,N,N)platinum (II) bromide)

The platinum complex (1b''-1) obtained in Example 5 ((6-phenyl-2,2'- bipyridinato-C,N,N)platinum (II) chloride) (500 mg, 1.08 mmol, 1.0 equivalent) and sodium bromide (811 mg, 5.42 mmol, 5.0 equivalents) were charged into a schlenk flask, whose inside was replaced with nitrogen. Then, 2-ethoxyethanol (10 mL) was added thereto, and the mixture was heated, and allowed to react with stirring under reflux for 3 hours. Then, the solvent was distilled off under reduced pressure. The residue was purified with a silica gel column chromatography (eluent: dichloromethane / methanol = 50/1). As a result, 447 mg of the platinum complex (1''-2) was obtained as a red purple crystal. Yield 81.5 %.
¹H-NMR (500MHz, CD₂Cl₂) :δ 7.09 (dt, J=1. 3, 7.6Hz, 1H), 7.15-7.21 (m, 1H), 7.38 (dd, J=1.4, 7.6Hz, 1H), 7.54-7.64(m, 2H), 7.66(d dd, J=1.3, 5.3, 7.7Hz, 1H), 7.75-7.96(m, 3H), 8.09(dt, J=1.7,7.9 Hz, 1H), 9.22-9.28 (m, 1H).

### Example 10 Production of platinum complex (1"-3) ((6-phenyl-2,2'-bipyridinato-C,N,N)platinum (II) iodide)

The platinum complex (1b''-1) obtained in the same manner as with Example 5 ((6-phenyl-2,2'-bipyridinato-C,N,N) platinum (II) chloride) (522 mg, 1.13mmol, 1.0 equivalent) and sodium iodide (847 mg, 5.65 mmol, 5.0 equivalents) were charged into a schlenk flask, whose inside was replaced with nitrogen. Then, 2-ethoxyethanol (10 mL) was added thereto, and the mixture was heated, and allowed to react with stirring under reflux for 3 hours. Then, the solvent was distilled off under reduced pressure. The residue was purified with a silica gel column chromatography (eluent: dichloromethane / methanol = 50/1), and then recrystallized from dichloromethane / diethyl ether. As a result, 561 mg of the platinum complex (1''-3) was obtained as an orange powder. Yield 89.7 %.
¹H-NMR(500MHz, CD₂Cl₂) :δ 7.05-7.14(m, 2H), 7.38(dd, J=1.7, 7.4Hz, 1H), 7.55-7.65(m, 3H), 7.94-8.00(m, 2H), 8.06(dt, J=1.6,7. 8Hz, 1H), 8.14-8.25(m, 1H), 9.54-9.60(m, 1H).
Excitation wavelength: 335.0 nm, Fluorescence wavelength: 561.4 nm.

### Example 11 Production of platinum complex (1''-3) (6-phenyl-2,2'-bipyridinato-C,N,N)platinum (II) iodide

[(1,2,5,6-η⁴)-1,5-hexadienyl]platinum(II) dichloride (300 mg, 0.862 mmol, 1.0 equivalent), 6-phenyl-2,2'-bipyridyl (240 mg, 1.034 mmol, 1.2 equivalents) and sodium iodide (646 mg, 4.310 mmol, 5.0 equivalents) were charged into a schlenk flask, whose inside was replaced with nitrogen. Then, 2-ethoxyethanol (6 mL) was added thereto, and the mixture was heated, and allowed to react with stirring under reflux for 3 hours. Then, the solvent was distilled off under reduced pressure. The resulting brown residue was purified with a silica gel column chromatography (eluent: dichloromethane /methanol = 50/1), and then recrystallized from dichloromethane / diethyl ether. As a result, 434 mg of the platinum complex (1''-3) was obtained as an orange powder. Yield 91.0 %. The physical property values of ¹H-NMR and the like were in accordance with those for the platinum complex obtained in Example 10.

### Example 12 Production of platinum complex (1"-3) ((6-phenyl-2,2'-bipyridinato-C,N,N)platinum (II) iodide) by a consecutive one pot method

[(1,2,5,6-η⁴)-1,5-hexadienyl]platinum(II) dichloride (300 mg, 0.862 mmol, 1.0 equivalent) and 6-phenyl-2,2'-bipyridyl (240 mg, 1.034 mmol, 1.2 equivalents) were charged into a schlenk flask, whose inside was replaced with nitrogen. Then, 2-ethoxyethanol (6 mL) was added thereto, and the mixture was heated, and allowed to react with stirring under reflux for 1 hour. Then, sodium iodide (646 mg, 4.310 mmol, 5.0 equivalents) was added thereto, and the resulting mixture was further stirred at the same temperature for 2 hours, and the solvent was distilled off under reduced pressure. The resulting orange residue was purified with a silica gel column chromatography (eluent: dichloromethane / methanol = 50/1), and then recrystallized from dichloromethane / diethyl ether. As a result, 468 mg of the platinum complex (1"-3) was obtained as an orange powder. Yield 98.1 %. The physical property values of ¹H-NMR and the like were in accordance with those for the platinum complex obtained in Example 10.

The results of Examples 10 to 12 indicate as follows. With the production method (Example 10) of the invention to be carried out in two stages, it is possible to produce a desirable platinum complex in a high yield. However, with the production method (Example 11) of the invention in which three of the platinum diene complex, the compound [3], and the halogenating agent are mixed, and allowed to mutually react, it is possible to obtain a desirable platinum complex in a higher yield. Further, with the production method (Example 12) of the invention to be carried out in one pot, it is possible to obtain a desirable platinum complex in a still higher yield.

### Example 13 Production of platinum complex (1"-7) ((6,6'-diphenyl-2,2'-bipyridinato-C,N,N)platinum (II) iodide)

The platinum complex (1b''-5) obtained in Example 6 ((6,6'-diphenyl-2,2'- bipyridinato-C,N,N)platinum (II) chloride) (300 mg, 0.558 mmol, 1.0 equivalent) and sodium iodide (418 mg, 2.790 mmol, 5.0 equivalents) were charged into a schlenk flask, whose inside was replaced with nitrogen. Then, 2-ethoxyethanol (15 mL) was added thereto, and the mixture was heated, and allowed to react with stirring under reflux for 6 hours. Then, the solvent was distilled off under reduced pressure. The residue was purified with a silica gel column chromatography (eluent: dichloromethane), and then, recrystallized from hexane / dichloromethane. As a result, 329 mg of the platinum complex (1''-7) was obtained as an orange powder. Yield 93.7 %.
¹H-NMR (500MHz, CD₂Cl₂) :δ 7.00-7.08(m, 2H), 7. 37-7. 42 (m, 1 H), 7.46-7.56(m, 3H), 7.60-7.67(m, 1H), 7.70-7.77(m, 3H), 7.78(dd, J=1.3, 7.8Hz, 1H), 8.02(dd, J=1.3, 7.9Hz, 1H), 8.04(t, J=7.9Hz, 1 H),8.10(t, J=7.8Hz, 1H), 8.17-8.34(m, 1H).

### Example 14 Production of platinum complex (1''-10) ((2,9-diphenyl-1,10-phenanthrolinato-C,N,N)platinum (II) iodide)

The platinum complex (1b''-9) obtained in Example 7 ((2,9-diphenyl-1,10-phenanthrolinato-C,N,N)platinum (II) chloride (300 mg, 0.534 mmol, 1.0 equivalent) and sodium iodide (400 mg, 2.670 mmol, 5.0 equivalents) were charged into a schlenk flask, whose inside was replaced with nitrogen. Then, 2-ethoxyethanol (15 mL) was added thereto, and the mixture was heated, and allowed to react with stirring under reflux for 3 hours. Then, the solvent was distilled off under reduced pressure. The residue was purified with a silica gel column chromatography (eluent: dichloromethane), and recrystallized from hexane / dichloromethane. As a result, 283 mg of the platinum complex (1"-10) was obtained as a red orange powder. Yield 81.1 %.
¹H-NMR (500MHz CD₂Cl₂) :δ 7.06-7.14 (m, 2H), 7.50-7.62 (m, 4 H),7.79-7.95(m,5H),7.98(d, J=8.4Hz,1H), 8.25-8.40(m,1H), 8.5 1(d, J=8.4Hz, 1H), 8.53(d, J=8.7Hz, 1H).
Excitation wavelength: 324 nm, Fluorescence wavelength: 587.4 nm.

### Example 15 Production of platinum complex (1b''-15) ((2,4,7,9-tetraphenyl-1,10-phenanthrolinato-C,N,N)platinum (II) iodide)

The platinum complex (1b''-15) obtained in Example 8 ((2,4,7,9-tetraphenyl-1, 10-phenanthrolinato-C,N,N)platinum (II) chloride (500 mg, 0.700 mmol, 1.0 equivalent) and sodium iodide (525 mg, 3.500 mmol, 5.0 equivalents) were charged into a schlenk flask, whose inside was replaced with nitrogen. Then, 2-ethoxyethanol (25 mL) was added thereto, and the mixture was heated, and allowed to react with stirring under reflux for 6 hours. Then, the solvent was distilled off under reduced pressure. The residue was purified with a silica gel column chromatography (eluent: dichloromethane), and then, recrystallized from hexane / dichloromethane. As a result, 541 mg of the platinum complex (1"-15) was obtained as a vermillion powder. Yield 95.9 %.
¹H-NMR(500MHz, CD₂Cl₂) :δ 7.06-7.14 (m, 2H), 7.52-7.67(m, 14 H), 7.85-7.93(m, 5H), 7.97(s, 1H), 8.26-8.42(m, 1H).
Excitation wavelength: 325 nm, Fluorescence wavelength: 614.2 nm.

### Example 16 Synthesis of platinum complex (1'''-2) ((6-phenyl-2, 2'-bipyridinato-(C,N,N) phenyl platinum (II))

### (1) Synthesis of [6-phenyl-2, 2'-bipyridinato-(C,N,N)] platinum (II) chloride

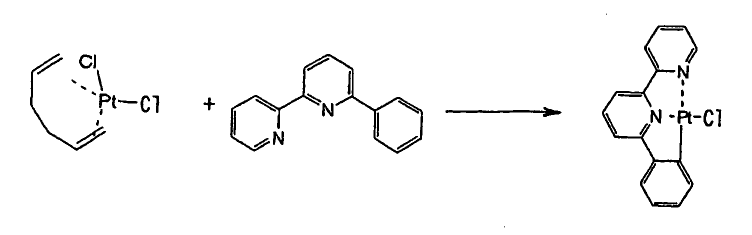

[(1,2,5,6-η⁴)-1,5-hexadienyl] platinum (II) dichloride (500 mg, 1.44 mmol) and 6-phenyl-2, 2'-bipyridyl (400 mg, 1.72 mmol) were charged into a schlenk flask, whose inside was replaced with nitrogen. Then, 2-ethoxyethanol (10 mL) was added thereto, and the mixture was allowed to react at 80 °C for 2 hours. Then, the solvent was distilled off under reduced pressure. The resulting red orange residue was purified with a silica gel column chromatography (eluent: dichloromethane / methanol = 20/1), and then, recrystallized from hexane /dichloromethane. As a result, 648 mg of the target material was obtained as an orange powder. Yield 97.4 %.
¹H-NMR (CD₂Cl₂) :δ 7.10 (dt, J=1.4, 7.5Hz, 1H), 7.16-7.24(m, 1H), 7.38(dd, J=1.4, 7.5Hz, 1H), 7.50-7.65(m, 3H), 7.68(ddd, J=1. 3Hz, 5.3, 7.9Hz, 1H), 7.88(t, J=8.0Hz, 1H), 7.94(dt, J=7.9,0.9Hz, 1H), 8.09(dt, J=1.6, 7.9Hz, 1H), 9.04-9.09(m, 1H)ppm.

### (2) Synthesis of platinum complex (1'''-2) ([6-phenyl-2, 2'-bipyridinato-(C,N,N)]phenyl platinum (II))

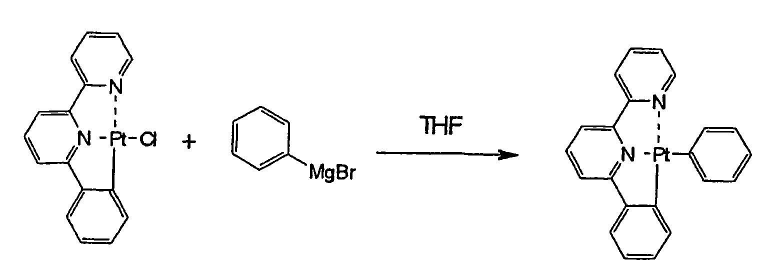

Into a Schlenk flask, [6-phenyl-2, 2'-bipyridinato-(C,N,N)] platinum (II) chloride obtained in the foregoing (1) (400 mg) was charged, and heated and dried under reduced pressure, followed by nitrogen replacement. 4 mL of tetrahydrofuran was added therein. Then, 1.67 mL (1.04 mol/L, 800 mmol) of a tetrahydrofuran solution of phenylmagnesium bromide was added dropwise at 20 °C. The mixture became a dark red solution. It was further stirred, so that an orange precipitate was gradually formed. The suspension was stirred at room temperature for 3 hours. Then, the solvent was distilled off under reduced pressure at 40 °C or less. To the resulting red residue, 25 mL of dichloromethane was added, followed by washing with 25 mL of water. The separated organic layer was purified with a silica gel column chromatography (eluent: dichloromethane). The fraction of the column was concentrated at 40 °C or less, and hexane was added thereto to perform crystallization. The crystal was obtained by filtration, and dried under reduced pressure. As a result, 375 mg of the platinum complex (1"'-2) of the invention was obtained as an orange powder.
Yield: 85.9 %
¹H-NMR(CD₂Cl₂)δ:6.97-7.19(m, 6H), 7.45-7.49(m, 2H), 7.57-7. 60(m, 1H), 7.65(dd, J=0.5, 8.2Hz, 1H), 7.68(dd, J=0.5, 7.9Hz, 1H), 7.85(t, J=8.0Hz, 1H), 7.95(dt, J=8.2, 0.9Hz, 1H), 8.03(dt, J=1. 6, 7.8Hz, 1H), 8.53-8.56(m, 1H)ppm.

### Example 17 Synthesis of platinum complex (1"'-1) [(6-phenyl-2- (2-pyridyl) pyridine (N,N,C)]pentafluorophenyl platinum (II))

### (1) Synthesis of dichloro(1,5-cyclooctadiene) platinum (II)

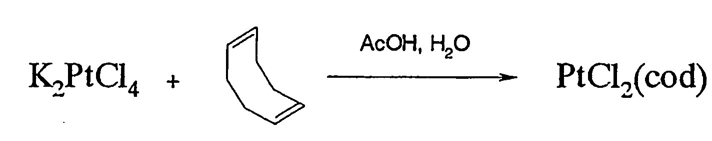

With reference to Jikken Kagaku Koza Fourth Edition (Fourth edition of Experimental Chemistry) (Maruzen), Vol. 18, Organic metal complex, p. 413, 1.0 g of potassium tetrachloroplatinate was dissolved in 16 mL of distilled water. 1 mL of 1, 5-cyclooctadiene and 24 mL of acetic acid were added thereto, and the mixture was stirred at 90 °C for 30 minutes. The precipitated creamy solid was obtained by filtration, and washed with water and methanol, followed by drying, resulting in 0.72 g of a creamy solid. Yield: 80.1 %
¹H-NMR(DMSO-D₆) δ:2.47-2.51(m, 8H), 5.23-5.52(m, 4H)ppm.

### (2) Synthesis of pentafluorophenyl(1,5-cyclooctadiene)platinum (II) chloride

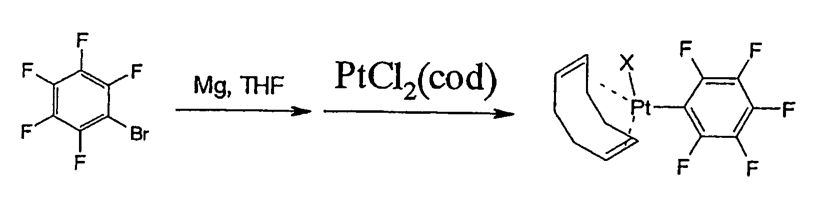

70 ml of a tetrahydrofuran solution of 1.2 g of pentafluorobromobenzene was added dropwise to 0.11 g of magnesium to prepare pentafluorophenylmagnesium bromide. 0.7 g of dichloro(1,5-cyclooctadiene)platinum (II) obtained in the foregoing (1) was added thereto, and the mixture was stirred at 20 °C for 2 hours. Water was added to the resulting yellow solution to terminate the reaction, and chloroform extraction was carried out. After concentration under reduced pressure, the residue was purified by a silica gel column chromatography (eluent: toluene / chloroform = 1 / 1). As a result, 0.784 g of the target material was obtained as a white solid. Yield: 82.8 %
¹H-NMR(CDCl3) δ:2.28-2.45(m, 4H), 2.61-2.72(m, 4H), 4.87-5. 01(m,2H),5.87-5.96(m,2H)ppm.

### (3) Synthesis of platinum complex (1"'-1) [(6-phenyl-2-(2-pyridyl)pyridine(N,N,C)]pentafluorophenyl platinum (II))

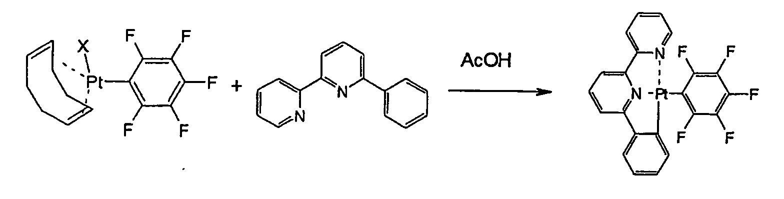

0.8 g of pentafluorophenyl(1,5-cyclooctadiene)platinum (II) chloride synthesized in the same manner as in the foregoing (2), 0.3 g of 6-phenyl-2-(2-pyridyl)pyridine, and 20 mL of acetic acid were charged, and the mixture was stirred at 80 °C for 2 days. The resulting orange solution was concentrated under reduced pressure, and purified by a silica gel column chromatography (eluent: toluene / chloroform = 1 / 1). As a result, 0.485 g of the platinum complex (1'''-1) was obtained as an orange solid. Yield: 51.7 %.
¹H-NMR(DMSO-D₆) δ: 6-67-6.80(m, 1H), 6.97(dt, J=1.5, 7.4Hz) , 7.03(dt, J=1.3, 7.3Hz), 7.65(dd, J=7.7, 1.4Hz), 7.68-7.71(m, 1H) , 8.04(d, J=8.0Hz, 1H), 8.18(t, J=8.0Hz, 1H), 8.25(dd, J=8.0, 0.7Hz ), 8.30-8.33(m, 2H), 8.52(dd, J=8.6, 1.1Hz, 1H)ppm.

### Example 18 Synthesis of platinum complex (1'''-2) ([6-phenyl-2,2'-bipyridinato-(C,N,N)]phenyl platinum (II))

### (1) Synthesis of [(6-phenyl-2,2'-bipyridinato-(C,N,N)]phenyl platinum (II))

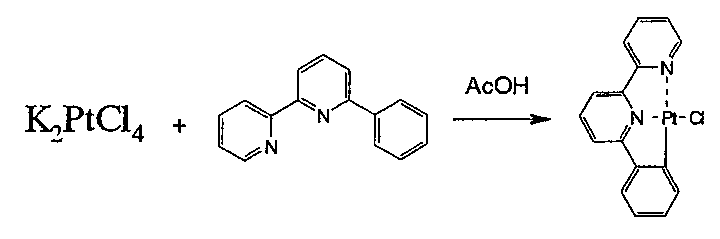

Into a Schlenk flask, were charged 1.0 g of potassium tetrachloroplatinate (II) and 0.56 g of 6-phenyl-2,2'-bipyridyl. 10 ml of acetic acid was added thereto, and the mixture was heated and allowed to react at 80 °C for 2 days. The reaction solution was cooled. The resulting red orange precipitate was obtained by filtration, and washed with water and methanol, followed by drying. As a result, 0.95 g of the target material was obtained as an orange material. Yield: 71.4 %
¹H-NMR(CD₂Cl₂) δ: 7.10 (dt, J=1.4, 7.5Hz, 1H), 7.16-7.24(m, 1H), 7.38(dd, J=1.4, 7.5Hz, 1H), 7.50-7.65(m, 3H), 7.68(ddd, J=1. 3Hz, 5.3, 7.9Hz, 1H), 7.88(t, J=8.0Hz, 1H), 7.94(dt, J=7.9,0.9Hz, 1H), 8.09(dt, J=1.6, 7.9Hz, 1H), 9.04-9.09(m, 1H)ppm.

### (2) Synthesis of platinum complex (1'''-2) [6-phenyl-2,2'-bipyridinato(C,N,N)]phenyl platinum (II))

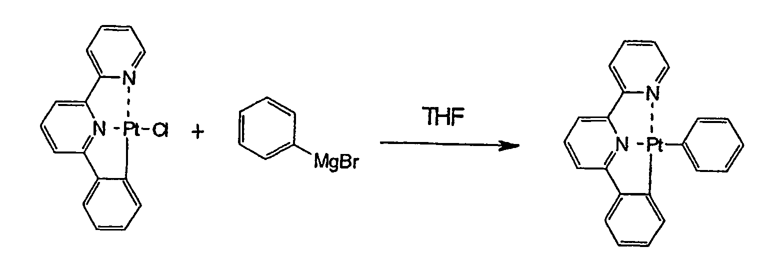

Into a Schlenk flask, were added 0.4 g of [6-phenyl-2,2'-bipyridinato-(C,N,N)] platinum (II) chloride obtained in the foregoing (1) and 4 ml of tetrahydrofuran. Then, 1.7 ml of a tetrahydrofuran solution (1.04 mol/L) of phenylmagnesium bromide was added dropwise thereto under a nitrogen atmosphere. The mixture was stirred at 20 °C for 3 hours, and then, the solvent was distilled off under reduced pressure. The resulting residue was subjected to dichloromethane extraction. It was purified by a silica gel column chromatography (eluent: dichloromethane). As a result, 370 mg of the platinum complex (1"'-1) was obtained as an orange solid. Yield: 84.8 %.

### Example 19 Synthesis of platinum complex (1"'-12) ([6-(2-naphthyl)-2,2'-pyridinato(N,N,C)]pentafluorophenyl platinum (II))

### (1) Synthesis of 6-(2-naphthyl)-2,2'-bipyridyl

The operation was carried out according to the method described in the documents (Chem. Ber., 109, 3864 to 3868 (1976), and Tetrahedoron Letter, 23, 5291 to 5294 (1982))

Into a Schlenk flask in which the inside atmosphere had been replaced with nitrogen, were charged 1.5 g of 2-bromonaphthalene and 30 ml of diethyl ether. At -50 °C, 4.5 ml of a hexane solution (1.6 mol/L) of n-butyl lithium was slowly added thereto. The mixture was stirred for 1 hour with the temperature kept, and further stirred for another hour by raising the temperature to 20 °C. Subsequently, 0.94 g of 2,2' -bipyridine was added thereto, and the mixture was stirred for 2 hours. The solution was extracted with diethyl ether, and the solvent was distilled off. The residue was oxidized with 600 ml of an acetone solution of 220 mg of potassium permanganate. The solution was filtered, and then, concentrated under reduced pressure, and purified by a silica gel column chromatography (eluent: toluene / ethyl acetate = 1/1). As a result, 0.7 g of the target material was obtained as a white powder. Yield: 41.2 %.
¹H-NMR(CD₂Cl₂) δ: 7.36-7.39(m, 1H), 7.52-7.56(m, 2H), 7.89-8. 02(m,6H),8.35(dd, J=9.6, 1.8Hz, 1H), 8.44(dd, J=6.4, 2.1Hz, 1H), 8.64(t, J=0.9Hz, 1H), 8.69-8.72(m, 2H)ppm.

### (2) Synthesis of platinum complex (1'''-12) ([6-(2-naphthyl)-2,2'-pyridinato(N,N,C)]pentafluorophenyl platinum (II))

To 0.23 g of pentafluorophenyl(1,5-cyclooctadiene)platinum (II) chloride synthesized in the same manner as with (2) of Example 17, and 0.59 g of 6-(2-naphthyl)-2,2'-bipyridine obtained in the foregoing (1), 20 ml of acetic acid was added. The mixture was stirred at 80 °C for 2 days. The resulting orange solution was concentrated under reduced pressure, and purified by a silica gel column chromatography (eluent: toluene / chloroform = 1 / 1). As a result, 0.04 g of the platinum complex (1"'-12) was obtained as an orange solid. Yield: 13.5 %.
¹H-NMR(CDCl3) δ:7.17(s, 1H), 7.23-7.31(m, 2H), 7.43-7.47(m, 2H), 7.67-7.70(m, 1H), 7.74(dd, J=7.9, 0.8Hz, 1H), 7.89(dd, J=8. 2, 0.8Hz, 1H), 7.95(s, 1H), 7.97-8.01(m, 2H), 8.06(dt, J=1.6,8.0H z, 1H), 8.30-8.36(m, 1H)ppm.

### Example 20 Synthesis of platinum complex (1'''-13) ([2,9-diphenyl phenanthrolinato (N,N,C)]pentafluorophenyl platinum (II))

To 0.23 g of pentafluorophenyl(1,5-cyclooctadiene)platinum (II) chloride synthesized in the same manner as with (2) of Example 17, and 0. 59 g of 2, 9-diphenylphenanthroline, 20 ml of acetic acid was added. The mixture was stirred at 80 °C for 2 days. The resulting orange solution was concentrated under reduced pressure, and purified by a silica gel column chromatography (eluent: toluene / chloroform = 1 / 1). As a result, 0.04 g of the platinum complex (1'''-12) was obtained as an orange solid. Yield: 12.7 %.
¹H-NMR(CD₂Cl₂) δ:6.70-6.53(m, 1H), 6.96(dt, J=1.4, 7.4Hz, 1 H),7.02(dt, J=1.3, 7.3Hz, 1H), 7.16-7.22(m, 3H), 7.45-7.47(m, 2 H),7.57(dd, J=7.6, 1.6Hz, 1H), 7.80(d, J=8.4Hz, 1H), 7.89-7.93 (m,2H),7.97(d, J=8.9Hz, 1H), 8.45(d, J=8.7Hz, 1H), 8.51(d, J=8. 4Hz,lH)ppm.

### Use Examples 1 to 3

An organic EL device of the structure shown in FIG. 1 was fabricated.

It is configured such that, on a glass substrate (g), an anode (ITO) (f), a hole transport layer (e), a light emitting layer (d) made of a host material and a dopant, a hole blocking layer (c), an electron transport layer (b), and a cathode (Al/LiF) (a) are formed sequentially from the glass substrate (g) side. To the anode (f) and the cathode (a), lead wires are respectively connected, which allows a voltage to be applied between the anode (f) and the cathode (a).

The anode (f) is an ITO film, and deposited on the glass substrate (g).

The hole transport layer (e) was formed with a thickness of 40 nm on the anode (f) by a vacuum deposition method using the following compound (α-NPD):

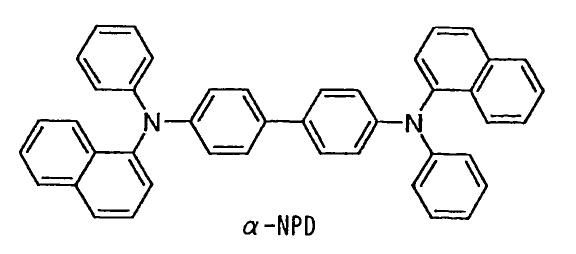

The light emitting layer (d) containing a host material and a doped phosphorescent material was formed with a thickness of 35 nm on the hole transport layer (e) simultaneously with a vacuum deposition method (dope 3 %) using both of the following compound (CBP): 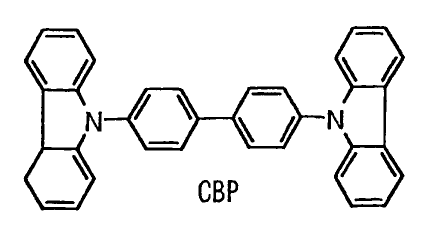 and the compound (1'-2) obtained in Example 2 (Use Example 1), the compound (1b''-1) obtained in Example 5 (Use Example 2), or the compound (1"'-1) obtained in Example 17 (Use Example 3).

The hole blocking layer (c) was formed with a thickness of 40 nm on the light emitting layer (d) by a vacuum deposition method using the following compound (BCP):

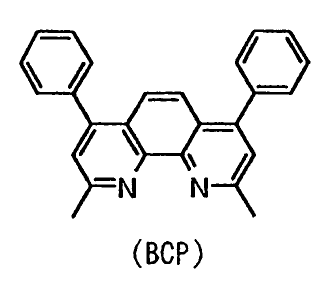

The electron transport layer (b) was formed with a thickness of 35 nm by a vacuum deposition method using Alq.

The cathode (a) was configured of a laminated member in which 0.5 nm LiF and 160 nm Al were formed from the electron transport layer (b) side. Incidentally, the degree of vacuum for vacuum depositing the respective layers of the organic compound layer and the cathode of each organic EL device in accordance with Use Examples 1 to 3 was 8 x 10⁻⁵ Pa.

Plus and minus voltages were applied to the anode (f) side and the cathode (a) side of the resulting organic EL device, respectively. As a result, the following results were shown:

i) When the compound (1'-2) was used (Use Example 1), stable luminescence was observed from at a very low voltage. Further, with an applied voltage of mere 5 V, a very high luminance of 200 cd/m² was achieved. The luminous quantum efficiency was a high efficiency of 8.3 % at a luminance of 100 cd/m². Further, green luminescence (luminous peak wavelength: 500 nm) caused by the compound (1'-2) used for the light emitting layer (d) was obtained.

Alternatively, ii) when the compound (1b''-1) is used (Use Example 2), stable yellow luminescence was observed from at a very low voltage. The spectrum of luminescence obtained from the organic EL device was in agreement with the photo-excited luminous spectrum of the thin film of the light emitting layer (d) alone composed of the platinum complex (1b''-1) and CBP vapor deposited on a quartz substrate. From this, it was shown that the luminescence caused by the platinum complex (1b''-1) used for the light emitting layer (d) was obtained from the organic EL device. Further, the thin film of the light emitting layer (d) alone was radiated with a pulse laser to analyze the change with time of the luminous intensity. As a result, the life in the excited state of the light emitting species was calculated to be 2.8 µs, and it was elucidated that the luminous phenomenon observed from the organic EL device in accordance with the invention was phosphorescence. The external quantum efficiency (which is calculated from the ratio of the number of photons emitted outside the device to the number of electric charges injected to the device) of the luminescence of the organic EL device was a high efficiency of 2.2 % at a luminance of 100 cd/m².

Further, iii) when the compound (1"'-1) is used (Use Example 3), as shown in Table 3, the luminous quantum efficiency of the device was a high efficiency of 3.2 % at a luminance of 100 cd/m². Further, yellow to orange luminescence (luminous peak wavelength: 554 nm) caused by the compound (1-1) used for the light emitting layer (d) was obtained.

The summary of the characteristics of the organic EL device fabricated in Use Example 1 using the compound (1'-2), and the summary of the characteristics of the organic EL device fabricated in Use Example 2 using the compound (1b'-1), and the summary of the characteristics of the organic EL device fabricated in Use Example 3 using the compound (1'''-1) are respectively shown in Table 1.

**Table 1**

| Use Example No. | Device structure | | | | Characteristics | | |
|---|---|---|---|---|---|---|---|
| | Hole transport layer | Light emitting layer | Hole blocking layer | Electron transport layer | CIE chromaticity point @100 cd/m² | External quantum efficiency @100 cd/m² (%) | Luminous efficiency @100 cd/m² (lm/W) |
| 1 | α-NPD | 3 % (1'-2) : CBP | BCP | Alq₃ | 0.26, 0.60 | 8.3 | 13 |
| 2 | α-NPD | 3 % (1b"-1) : CBP | BCP | Alq₃ | 0.49, 0.50 | 2.2 | 2.5 |
| 3 | α-NPD | 3 % (1"'-1) : CBP | BCP | Alq₃ | 0.48, 0.51 | 3.2 | 4.3 |

### Use Examples 4 to 6

The devices of Use Examples 4 and 5 were fabricated in the same manner as with Use Example 1, except that the compound (1'-1) obtained in Example 1, or the compound (1'-3) obtained in Example 3 was used in place of the compound (1'-2) in Use Example 1. Further, the device of Use Example 6 was fabricated in the same manner using the compound (1'-3) in an amount of 6 %. These EL characteristics were evaluated.

The the characteristics of the organic EL device fabricated in Use Examples 4 to 6 are summarized and shown in Table 2 below.

**Table 2**

| Use Example No. | Device structure | | | | Characteristics | | |
|---|---|---|---|---|---|---|---|
| | Hole transport layer | Light emitting layer | Hole blocking layer | Electron transport layer | CIE chromaticity point @100 cd/m² | External quantum efficiency @100 cd/m² (%) | Luminous efficiency @100 cd/m² (lm/W) |
| 4 | α-NPD | 3 % (1'-1) : CBP | BCP | Alq₃ | 0.26, 0.60 | 6.6 | 11.1 |
| 5 | α-NPD | 3 % (1'-3) : CBP | BCP | Alq₃ | 0.27, 0.60 | 5.8 | 6.1 |
| 6 | α-NPD | 6 % (1'-3) : CBP | BCP | Alq₃ | 0.28, 0.60 | 5.9 | 6.3 |

### Use Examples 7 to 11

A device of Use Example 7 which has the same device structure as with Use Example 2, and in which the dope amount of the platinum complex (1b"-1) in the light emitting layer (d) had been set at 6 % was fabricated. Whereas, devices of Use Examples 8 and 9 each of which has the same device structure as with Use Example 2, and in which the platinum complex (1''-3) obtained in Example 10 had been doped in amounts of 3 % and 6 % in the light emitting layer (d), respectively, were fabricated. Further, a device of Use Example 10 which has the same device structure as with Use Example 2, and in which the platinum complex (1''-10) obtained in Example 14 had been doped in an amount of 3 % in the light emitting layer (d), and a device of Use Example 11 in which the platinum complex (1''-15) obtained in Example 15 had been doped in an amount of 3 % were fabricated.

The characteristics of these devices were evaluated.

The characteristics of the organic EL devices fabricated in Use Examples 7 to 11 are summarized and shown in Table 3 below.

**Table 3**

| Use Example No. | Light emitting layer | CIE chromaticity point @100 cd/m² | External quantum efficiency @100 cd/m² (%) | Luminous efficiency @100 cd/m² |
|---|---|---|---|---|
| 7 | 6 % (1b"-1) : CBP | 0.49, 0.50 | 2.1 | 2.5 |
| 8 | 3 % (1"-3) : CBP | 0.50, 0.49 | 4.3 | 6.8 |
| 9 | 6 % (1"-3): CBP | 0.51, 0.49 | 4.4 | 6.8 |
| 10 | 3 % (1"-10) : CBP | 0.64, 0.36 | 6.4 | 3.1 |
| 11 | 3 % (1"-15) : CBP | 0.67, 0.33 | 4.1 | 1.0 |

As apparent from the foregoing results, it is shown that the organic EL device using the platinum complex in accordance with the invention shows a very excellent characteristics.

### Industrial Applicability

The platinum complex in accordance with the invention is useful as a phosphorescent material, and applicable to the fabrication of various display devices, particularly, a high efficiency organic EL device.

Further, in accordance with the production method of the invention, it is possible to manufacture the platinum complex in accordance with the invention with ease and in a very high yield.

## Claims

1. A platinum complex represented by the following general formula [1]: 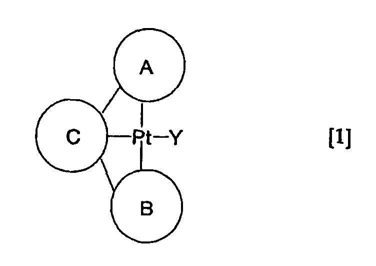 [where in the formula, any two of a ring A, a ring B, and a ring C each independently represent a nitrogen-containing aromatic heterocyclic group coordinating to a platinum atom through a nitrogen atom, which may be substituted, and the other represents an aryl group which may be substituted, or a heteroaryl group which may be substituted, and Y represents a halogen atom, or an aryl group which may be substituted, or a heteroaryl group which may be substituted, bonded directly or through an oxygen atom (-O-) or a sulfur atom (-S-) (provided that when the adjacent two rings are nitrogen-containing aromatic heterocyclic groups, the cases where Y is a chlorine atom are excluded, and that when the nonadjacent two rings are nitrogen-containing aromatic heterocyclic groups, the cases where Y is a group other than a halogen atom are excluded)].

2. The platinum complex according to claim 1, represented by the following general formula [1']: 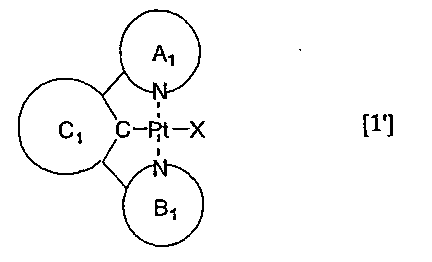 (where in the formula, a ring A₁ and a ring B₁ each independently represent a nitrogen-containing aromatic heterocyclic group which may be substituted, a ring C₁ represents an aryl group which may be substituted or a heteroaryl group which may be substituted, and X represents a halogen atom.)

3. The platinum complex according to claim 2, represented by the following general formula [1a']: 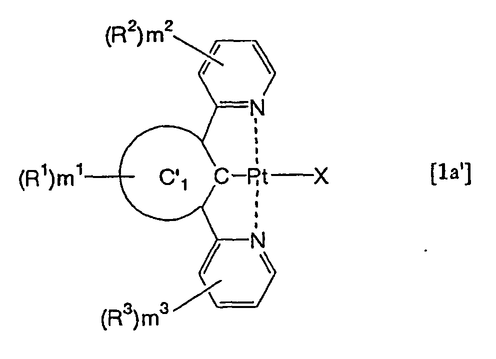 (where in the formula, a ring C'₁ represents an aryl group or a heteroaryl group; R¹, R², and R³ each independently represent a hydrogen atom, an alkyl group, a haloalkyl group, an aralkyl group, an alkenyl group, an alkynyl group, an aryl group, an amino group, a mono- or di-alkylamino group, a mono- or di-arylamino group, an alkoxy group, an aryloxy group, a heteroaryloxy group, an alkoxycarbonyl group, an acyloxy group, an acylamino group, an alkoxycarbonylamino group, an aryloxycarbonylamino group, a sulfonylamino group, a sulfamoyl group, a carbamoyl group, an alkylthio group, an arylthio group, a heteroarylthio group, a sulfonyl group, a sulfinyl group, an ureido group, a phosphoramide group, a hydroxyl group, a mercapto group, a halogen atom, a cyano group, a sulfo group, a carboxyl group, a nitro group, a hydroxamic acid group, a sulfino group, a hydrazino group, an aliphatic heterocyclic group, an aromatic heterocyclic group, a substituted silyl group, or a polymerizable group; further, a plurality of R¹s, a plurality of R²s, or/and a plurality of R³s may together form a fused ring with pyridine rings or the ring C to which they are bonded; X represents a halogen atom; m¹, m², and m³ represent numbers of the substituents R¹, R², and R³, respectively, and m¹ represents an integer of 0 to 3, and m² and m³ each represent an integer of 0 to 4; and further, when m¹, m², and m³ each is an integer of 2 or more, a plurality of R¹s, a plurality of R²s, and a plurality of R³s may be mutually the same or different.)

4. The platinum complex according to claim 2 or 3, represented by the following general formula [1b']: 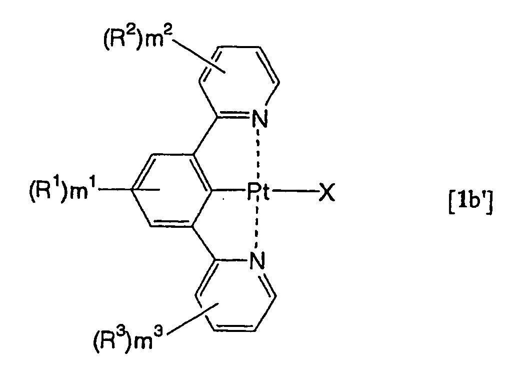 (where in the formula,, R¹, R², R³, X, m¹, m², and m³ represent the same meanings as described above.)

5. The platinum complex according to claim 1, represented by the following general formula [1'']: 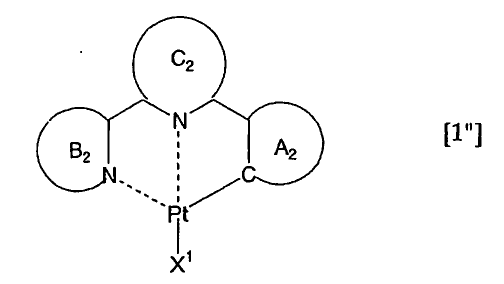 (where in the formula, a ring B₂ and a ring C₂ each independently represent a nitrogen-containing aromatic heterocyclic group which may be substituted, a ring A₂ represents an aryl group which may be substituted or a heteroaryl group which may be substituted; further, the ring B₂ and the ring C₂, the ring C₂ and the ring A₂, or the ring B₂, the ring C₂, and the ring A₂ may be mutually bonded to form a fused ring; and X¹ represents a fluorine atom, a bromine atom, or an iodine atom).

6. The platinum complex according to claim 5, represented by the following general formula [1a'']: 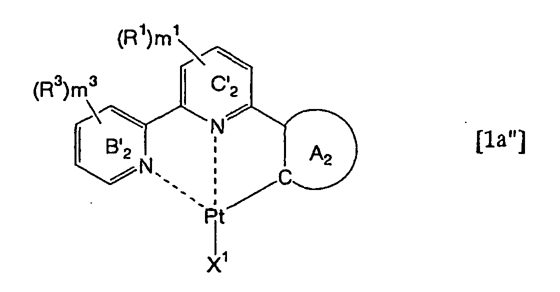 (where in the formula, a ring A₂ represents an aryl group which may be substituted or a heteroaryl group which may be substituted; R¹ and R³ each independently represent a hydrogen atom, an alkyl group, a haloalkyl group, an aralkyl group, an alkenyl group, an alkynyl group, an aryl group, an amino group, a mono- or di-alkylamino group, a mono- or di-arylamino group, an alkoxy group, an aryloxy group, a heteroaryloxy group, an alkoxycarbonyl group, an acyloxy group, an acylamino group, an alkoxycarbonylamino group, an aryloxycarbonylamino group, a sulfonylamino group, a sulfamoyl group, a carbamoyl group, an alkylthio group, an arylthio group, a heteroarylthio group, a sulfonyl group, a sulfinyl group, an ureido group, a phosphoramide group, a hydroxyl group, a mercapto group, a halogen atom, a cyano group, a sulfo group, a carboxyl group, a nitro group, a hydroxamic acid group, a sulfino group, a hydrazino group, an aliphatic heterocyclic group, an aromatic heterocyclic group, a substituted silyl group, or a polymerizable group; further, a plurality of R¹s or/and a plurality of R³s may together form a fused ring with pyridine rings to which they are bonded; further, the ring B'₂ and the ring C'₂, and C'₂ and the ring A₂, or the ring B'₂, the ring C'₂, and the ring A₂ may be mutually bonded to form a fused ring; m¹ and m³ represent the numbers of the substituents R¹ and R³, respectively, and m¹ represents an integer of 0 to 3, and m³ represents an integer of 0 to 4; and further, when m¹ and m³ are each an integer of 2 or more, a plurality of R¹s and a plurality of R³s may be mutually the same or different; and X¹ is the same as described above).

7. The platinum complex according to claim 1, represented by the following general formula [1''']: 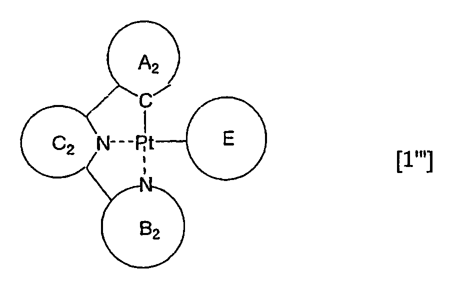 (where in the formula, a ring B₂ and a ring C₂ each independently represent a nitrogen-containing aromatic heterocyclic group which may be substituted, a ring A₂ and a ring E each independently represent an aryl group which may be substituted, or a heteroaryl group which may be substituted; the ring A₂ and the ring C₂, and the ring C₂ and the ring B₂, or the ring A₂, the ring C₂, and the ring B₂ may be mutually bonded to form a fused ring; further, in the case where the ring A₂, the ring B₂, the ring C₂, or/and the ring E each have a substituent, when the substituent is a substituent capable of coordinating or bonding to a metal, a metal atom may be coordinated or bonded through a coordinatable or bondable atom in the substituent.)

8. The platinum complex according to claim 7, represented by the following general formula [1a''']: 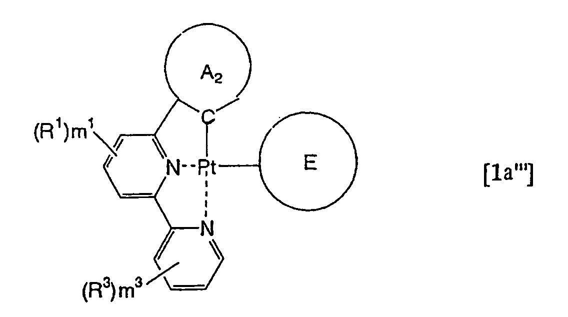 (where in the formula, the ring A₂ and the ring E are the same as described above; R¹ and R³ each independently represent an alkyl group, a haloalkyl group, an aralkyl group, an alkenyl group, an alkynyl group, an aryl group, an amino group, a mono- or di-alkylamino group, a mono- or di-arylamino group, an alkoxy group, an aryloxy group, a heteroaryloxy group, an alkoxycarbonyl group, an aryloxycarbonyl group, an acyloxy group, an acylamino group, an alkoxycarbonylamino group, an aryloxycarbonylamino group, a sulfonylamino group, a sulfamoyl group, a carbamoyl group, an alkylthio group, an arylthio group, a heteroarylthio group, a sulfonyl group, a sulfinyl group, an ureido group, a phosphoramide group, a hydroxyl group, a mercapto group, a halogen atom, a cyano group, a sulfo group, a carboxyl group, a nitro group, a hydroxamic acid group, a sulfino group, a hydrazino group, an aliphatic heterocyclic group, an aromatic heterocyclic group, a substituted silyl group, or a polymerizable group; further, R¹ and R³ may together form a fused ring with two pyridine rings to which they are bonded; R¹ and the ring A₂, or R¹, R³, and the ring A₂ may together form a fused ring; m¹ and m³ represent the numbers of the substituents R¹ and R³, respectively, m¹ represents an integer of 0 to 3, and m³ represents an integer of 0 to 4; and further, when m¹ and m³ are each an integer of 2 or more, a plurality of R¹s and a plurality of R³s may be mutually the same or different; further, a plurality of R¹s or/and a plurality of R³s may together form a fused ring with the pyridine rings to which they are bonded; and further when R¹, R³, and each substituent in the ring A₂ or/and the ring E are each a substituent capable of coordinating to a metal or capable of bonding to a metal, a metal atom may be coordinated or bonded through a coordinatable or bondable atom in the substituent.)

9. The platinum complex according to claim 7 or 8, represented by the following general formula [1b''']: 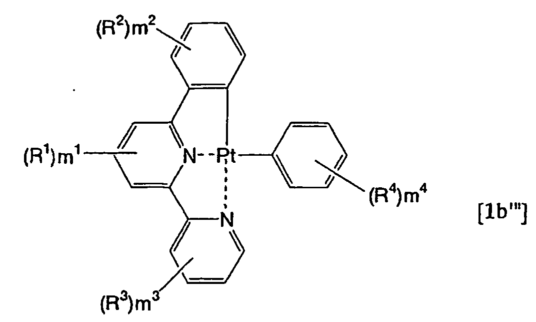 (where in the formula, R¹, R², R³, and R⁴ each independently represent an alkyl group, a haloalkyl group, an aralkyl group, an alkenyl group, an alkynyl group, an aryl group, an amino group, a mono- or di-alkylamino group, a mono- or di-arylamino group, an alkoxy group, an aryloxy group, a heteroaryloxy group, an alkoxycarbonyl group, an aryloxycarbonyl group, an acyloxy group, an acylamino group, an alkoxycarbonylamino group, an aryloxycarbonylamino group, a sulfonylamino group, a sulfamoyl group, a carbamoyl group, an alkylthio group, an arylthio group, a heteroarylthio group, a sulfonyl group, a sulfinyl group, an ureido group, a phosphoramide group, a hydroxyl group, a mercapto group, a halogen atom, a cyano group, a sulfo group, a carboxyl group, a nitro group, a hydroxamic acid group, a sulfino group, a hydrazino group, an aliphatic heterocyclic group, an aromatic heterocyclic group, a substituted silyl group, or a polymerizable group; further, R¹ and R², R¹ and R³, or/and R¹, R², and R³ may together form a fused ring with the two pyridine rings, or the pyridine ring and the benzene ring to which they are bonded; m¹, m², m³, and m⁴ represent the numbers of the substituents R¹, R², R³, and R⁴, respectively, m¹ represents an integer of 0 to 3, m² and m³ each represent an integer of 0 to 4, and m⁴ represents an integer of 0 to 5; and further, when m¹, m², m³, and m⁴ are each an integer of 2 or more, a plurality of R¹s, a plurality of R²s, a plurality of R³s, and a plurality of R⁴ may be mutually the same or different; further, R¹s, R²s, R³s, or/and R⁴s may together form a fused ring with the pyridine rings or the benzene rings to which they are bonded; and further when R¹, R², R³, and/or R⁴ are each a substituent capable of coordinating to a metal or capable of bonding to a metal, a metal atom may be coordinated or bonded through a coordinatable or bondable atom in the substituent.)

10. A method for producing the platinum complex according to claim 5, **characterized by** allowing a platinum diene complex represented by the general formula [2]:
Pt(X¹)₂(D) [2]
(where in the formula, D represents a nonconjugated diene compound, and X¹ represents a fluorine atom, a bromine atom, or an iodine atom) and a compound represented by the general formula [3]: 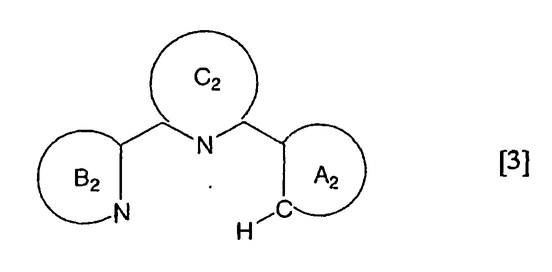 (where in the formula, the ring B₂ and the ring C₂ each independently represent a nitrogen-containing aromatic heterocyclic group which may be substituted, the ring A₂ represents an aryl group which may be substituted or a heteroaryl group which may be substituted, and the ring B₂ and the ring C₂, the ring C₂ and the ring A₂, or the ring B₂, the ring C₂, and the ring A₂ may be mutually bonded to form a fused ring) to react with each other.

11. A method for producing the platinum complex according to claim 5, **characterized by** allowing a platinum diene complex represented by the general formula [2a]:
Pt(Cl)₂(D) [2a]
(where in the formula, D represents a nonconjugated diene compound), a compound represented by the general formula [3] : 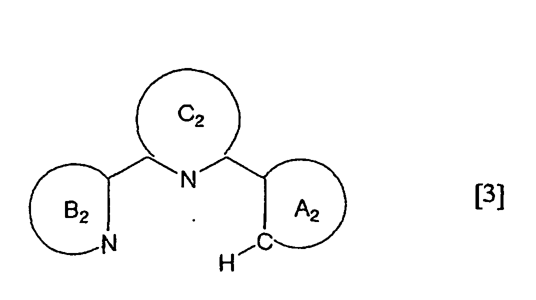 (where in the formula, the ring B₂ and the ring C₂ each independently represent a nitrogen-containing aromatic heterocyclic group which may be substituted, the ring A₂ represents an aryl group which may be substituted or a heteroaryl group which may be substituted, and the ring B₂ and the ring C₂, the ring C₂ and the ring A₂, or the ring B₂, the ring C₂, and the ring A₂ may be mutually bonded to form a fused ring), and a halogenating agent for substituting a halogen atom other than chlorine to mutually react.

12. The production method according to claim 11, wherein the platinum diene complex represented by the general formula [2a] is first allowed to react with the compound represented by the general formula [3], and then, allowed to react with the halogenating agent for substituting a halogen atom other than chlorine.

13. The production method according to claim 12, wherein the reaction of the platinum diene complex represented by the general formula [2a] and the compound represented by the general formula [3], and the subsequent reaction with the halogenating agent are carried out in one pot.

14. A method for producing the platinum complex according to claim 5, **characterized by** allowing a platinum complex represented by the general formula [1b'']: 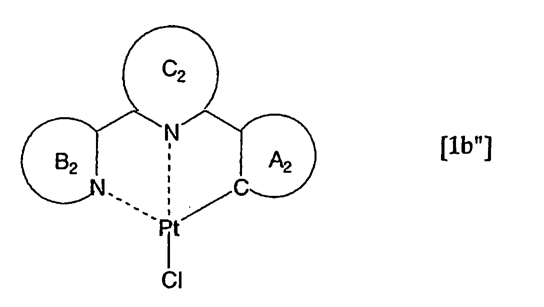 (where in the formula, the ring B₂ and the ring C₂ each independently represent a nitrogen-containing aromatic heterocyclic group which may be substituted, the ring A₂ represents an aryl group which may be substituted or a heteroaryl group which may be substituted, and the ring B₂ and the ring C₂, the ring C₂ and the ring A₂, or the ring B₂, the ring C₂, and the ring A₂ may be mutually bonded to form a fused ring), and a halogenating agent for substituting a halogen atom other than chlorine to react with each other.

15. A method for producing a platinum complex having a tridentate ligand, and having a halogen atom, **characterized by** using a platinum diene complex represented by the general formula [2b]:
Pt(X)₂(D) [2b]
(where in the formula, D represents a nonconjugated diene compound, and X represents a halogen atom) as a platinum source.

16. The production method according to claim 15, wherein the platinum complex having a tridentate ligand, and having a halogen atom is a platinum complex represented by the following general formula [1c'']: 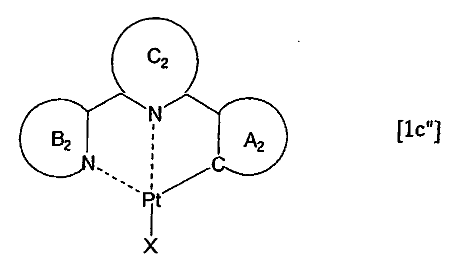 (where in the formula, the ring B₂ and the ring C₂ each independently represent a nitrogen-containing aromatic heterocyclic group which may be substituted, the ring A₂ represents an aryl group which may be substituted or a heteroaryl group which may be substituted, and the ring B₂ and the ring C₂, the ring C₂ and the ring A₂, or the ring B₂, the ring C₂, and the ring A₂ may be mutually bonded to form a fused ring; and X represents a halogen atom).

17. A method for producing a platinum complex represented by the general formula [1c'']: 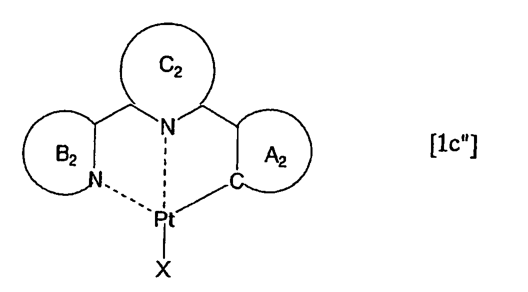 (where in the formula, the ring B₂, the ring C₂, the ring A₂, and X are the same as described above.), **characterized by** allowing a platinum diene complex represented by the general formula [2b]:
Pt(X)₂(D) [2b]
(where in the formula, D represents a nonconjugated diene compound, and X represents a halogen atom.) and a compound represented by the general formula [3]: 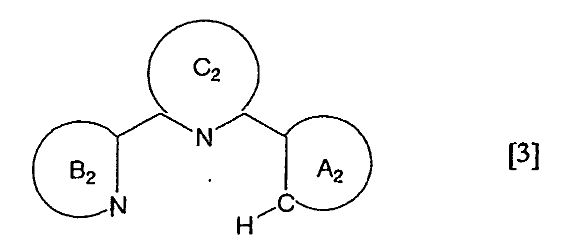 (where in the formula, the ring B₂ and the ring C₂ each independently represent a nitrogen-containing aromatic heterocyclic group which may be substituted, the ring A₂ represents an aryl group which may be substituted or a heteroaryl group which may be substituted, and the ring B₂ and the ring C₂, the ring C₂ and the ring A₂, or the ring B₂, the ring C₂, and the ring A₂ may be mutually bonded to form a fused ring) to react with each other.

18. A method for producing a platinum complex represented by the general formula [1d'']: 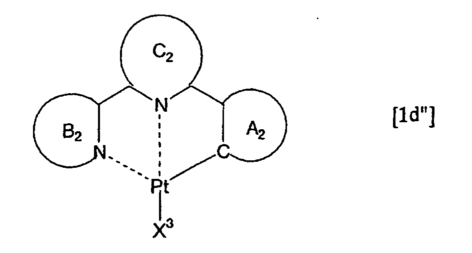 (where in the formula, the ring B₂, the ring C₂, and the ring A₂ are the same as described above; and X³ represents a halogen atom (provided that the cases where X and X³ are the same are excluded)), **characterized by** allowing a platinum diene complex represented by the general formula [2b]:
Pt(X)₂(D) [2b]
(where in the formula, D represents a nonconjugated diene compound, and X represents a halogen atom), a compound represented by the general formula [3]: 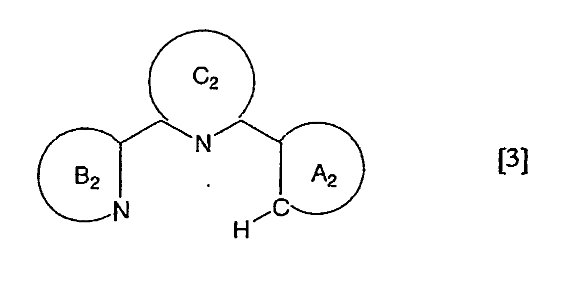 (where in the formula, the ring B₂ and the ring C₂ each independently represent a nitrogen-containing aromatic heterocyclic group which may be substituted, the ring A₂ represents an aryl group which may be substituted or a heteroaryl group which may be substituted, and the ring B₂ and the ring C₂, the ring C₂ and the ring A₂, or the ring B₂, the ring C₂, and the ring A₂ may be mutually bonded to form a fused ring), and a halogenating agent for substituting a halogen atom other than X to mutually react.

19. The production method according to claim 18, wherein the platinum diene complex represented by the general formula [2b] is first allowed to react with the compound represented by the general formula [3], and then, allowed to react with the halogenating agent for substituting a halogen atom other than X.

20. The production method according to claim 19, wherein the reaction of the platinum diene complex represented by the general formula [2b] and the compound represented by the general formula [3], and the subsequent reaction with the halogenating agent are carried out in one pot.

21. A method for producing a platinum complex represented by the general formula [1d'']: 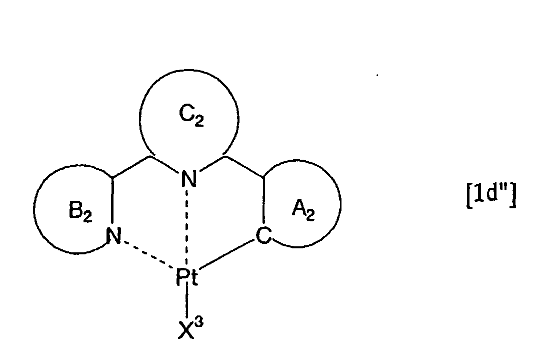 (where in the formula, the ring B₂, the ring C₂, and the ring A₂ are the same as described above; and X³ represents a halogen atom (provided that the cases where X and X³ are the same are excluded)), **characterized by** allowing a platinum complex represented by the general formula [1c'']: 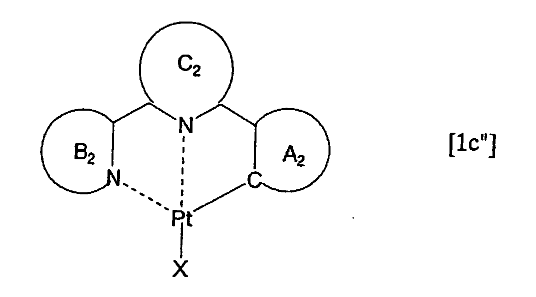 (where in the formula, the ring B₂ and the ring C₂ each independently represent a nitrogen-containing aromatic heterocyclic group which may be substituted, the ring A₂ represents an aryl group which may be substituted or a heteroaryl group which may be substituted, and the ring B₂ and the ring C₂, the ring C₂ and the ring A₂, or the ring B₂, the ring C₂, and the ring A₂ may be mutually bonded to form a fused ring; and X represents a halogen atom), and a halogenating agent for substituting a halogen atom other than X to react with each other.

22. A method for producing the platinum complex according to claim 7, **characterized by** allowing a platinum diene complex represented by the general formula [2b]:
Pt(X)₂(D) [2b]
(where in the formula, D represents a nonconjugated diene compound, and X represents a halogen atom) and a compound represented by the general formula [3]: 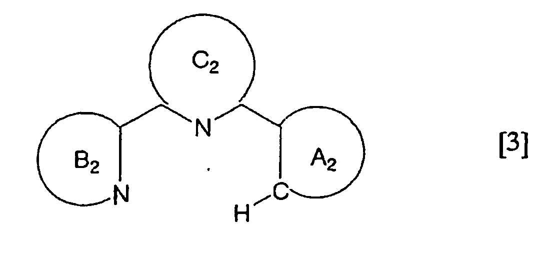 (where in the formula, the ring B₂ and the ring C₂ each independently represent a nitrogen-containing aromatic heterocyclic group which may be substituted, the ring A₂ represents an aryl group which may be substituted or a heteroaryl group which may be substituted, and the ring B₂ and the ring C₂, the ring C₂ and the ring A₂, or the ring B₂, the ring C₂, and the ring A₂ may be mutually bonded to form a fused ring) to react with each other, and thereby forming a platinum complex represented by the general formula [1c'']: 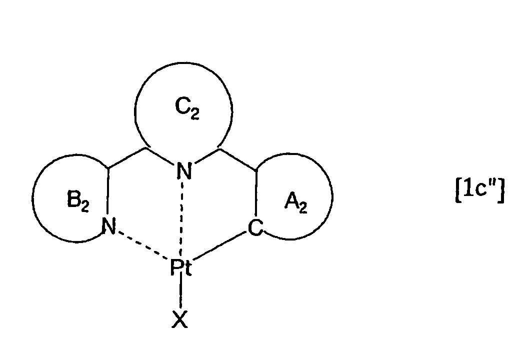 (where in the formula, the ring B₂, the ring C₂, the ring A₂, and X are the same as described above), and then, allowing a Grignard reagent represented by the general formula [4]:
EMgX² [4]
(where in the formula, E represents an aryl group which may be substituted or a heteroaryl group which may be substituted, and X² represents a halogen atom) to act thereon.

23. A method for producing the platinum complex according to claim 7, **characterized by** allowing a platinum compound represented by the general formula [5]:
M₂PtX₄ [5]
(where in the formula, M represents an alkali metal atom, and X represents a halogen atom.)) and a compound represented by the general formula [3]: 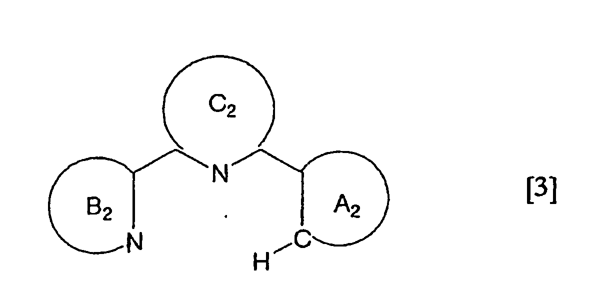 (where in the formula, the ring B₂, the ring C₂, and the ring A₂ are the same as described above) to react with each other, and thereby forming a platinum complex represented by the general formula [1c'']: 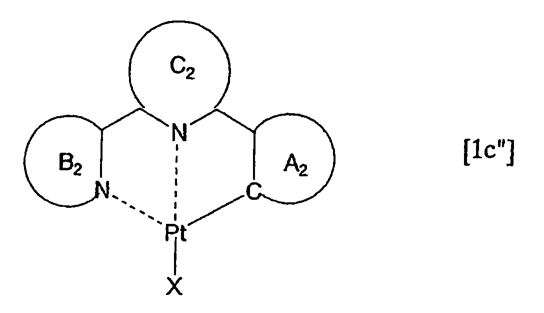 (where in the formula, the ring B₂, the ring C₂, the ring A₂, and X are the same as described above), and then, allowing a Grignard reagent represented by the general formula [4]:
EMgX² [4]
(where in the formula, E represents an aryl group which may be substituted or a heteroaryl group which may be substituted, and X² represents a halogen atom) to act thereon.

24. A method for producing the platinum complex according to claim 7, **characterized by**, on a platinum diene complex represented by the general formula [2b]:
Pt(X)₂(D) [2b]
(where in the formula, D represents a nonconjugated diene compound, and X represents a halogen atom), allowing a Grignard reagent represented by the general formula [4]:
EMgX² [4]
(where in the formula, E represents an aryl group which may be substituted or a heteroaryl group which may be substituted, and X² represents a halogen atom) to act, and then, allowing a compound represented by the general formula [3]: 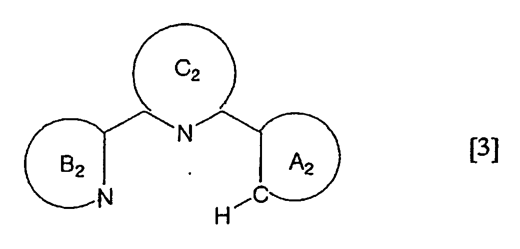 (where in the formula, the ring B₂ and the ring C₂ each independently represent a nitrogen-containing aromatic heterocyclic group which may be substituted, the ring A₂ represents an aryl group which may be substituted or a heteroaryl group which may be substituted, and the ring B₂ and the ring C₂, the ring C₂ and the ring A₂, or the ring B₂, the ring C₂, and the ring A₂ may be mutually bonded to form a fused ring) to react therewith.
